# EUROPEAN PATENT SPECIFICATION

(11) **EP 1 830 842 B1**
(45) Date of publication and mention of the grant of the patent: **22.04.2009**
(21) Application number: 05823645.6
(22) Date of filing: 21.12.2005
(51) Int. Cl.: A61K 31/404

(54) **MEDICAMENTS FOR THE TREATMENT OR PREVENTION OF FIBROTIC DISEASES**
MEDIKAMENTE ZUR BEHANDLUNG ODER PRÄVENTION FIBROTISCHER KRANKHEITEN
MEDICAMENTS POUR LE TRAITEMENT OU LA PREVENTION DE MALADIES FIBROTIQUES

(30) Priority: 24.12.2004 EP 04030768
(43) Date of publication of application: 12.09.2007
(73) Proprietor: Boehringer Ingelheim International GmbH, 55216 Ingelheim am Rhein (DE); Boehringer Ingelheim Pharma GmbH & Co.KG, 55216 Ingelheim am Rhein (DE)
(72) Inventor: PARK, John, Edward, 88447 Warthausen (DE); CHAUDHARY, Nveed, Wembley, Middlesex HA9 8DG (GB); LEHMANN-LINTZ, Thorsten, 88416 Ochsenhausen (DE); HECKEL, Armin, 88400 Biberach (DE); ROTH, Gerald, Jürgen, 88400 Biberach (DE); KLEY, Jörg, 88441 Mittelbiberach (DE); BRANDL, Trixi, 4056 Basel (CH); DAHMANN, Georg, 88448 Attenweiler (DE); GRAUERT, Matthias, 88400 Biberach (DE)
(74) Representative: Hammann, Heinz
(86) International application number: PCT/EP2005/057006
(87) International publication number: WO 2006/067168

(56) References cited:
- WO-A-02/081445

## Description

The present invention relates to a new use of indolinones of general formula 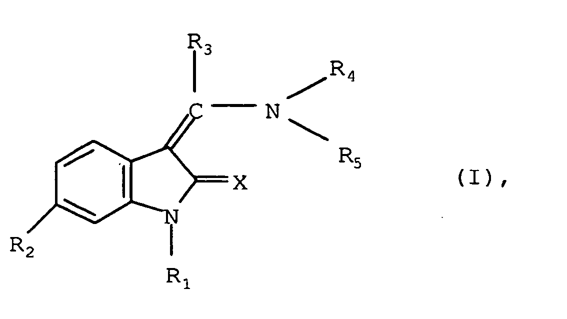 substituted in the 6 position, the tautomers, the diastereomers, the enantiomers, the mixtures thereof and the salts thereof, particularly the physiologically acceptable salts thereof.

### BACKGROUND

Compounds of the above general formula I, the tautomers, the diastereomers, the enantiomers, the mixtures thereof and the salts thereof, particularly the physiologically acceptable salts thereof, have been described in WO 02/81445 as having valuable pharmacological properties, in particular an inhibiting effect on various kinases, especially receptor tyrosine kinases such as VEGFR2, VEGFR3, PDGFRα, PDGFRβ, FGFR1, FGFR3, EGFR, HER2, IGF1R and HGFR, as well as complexes of CDK's (Cyclin Dependent Kinases) such as CDK1, CDK2, CDK3, CDK4, CDK5, CDK6, CDK7, CDK8 and CDK9 with their specific cyclins (A, B1, B2, C, D1, D2, D3, E, F, G1, G2, H, I and K) and to viral cyclin (cf. L. Mengtao in J. Virology 71(3), 1984-1991 (1997)), and on the proliferation of cultivated human cells, in particular endothelial cells, e.g. in angiogenesis, but also on the proliferation of other cells, in particular tumour cells.

However, none of these compounds have been described for their use in the treatment or prevention of the fibrotic diseases referred to in the present invention.

Remodeling is a normal response to tissue injury and inflammation that is observed in many tissues throughout the body. After resolution of the inflammation and repair of tissue damage, the tissue is generally returned to its original condition. Excessive uncontrolled tissue repair or the failure to stop remodeling when it is no longer required leads to condition known as fibrosis. Fibrosis is characterized by excessive deposition of extracellular matrix components and overgrowth of fibroblasts. Fibrosis can occur in all tissues but is especially prevalent in organs with frequent exposure to chemical and biological insults including the lung, skin, digestive tract, kidney, and liver (Eddy, 1996, J Am Soc Nephrol, 7(12):2495-503; Dacic et al., 2003, Am J Respir Cell Mol Biol, 29S: S5-9; Wynn, 2004, Nat Rev Immunol, 4 (8):583-94). Fibrosis often severely compromises the normal function(s) of the organ and many fibrotic diseases are, in fact, life-threatening or severely disfiguring, such as idiopathic pulmonary fibrosis (IPF), liver cirrhosis, scleroderma, or renal fibrosis. Treatment options for these diseases are often limited to organ transplantation, a risky and expensive procedure.

A large body of literature implicates the platelet-derived growth factor (PDGF), fibroblast growth factor (FGF), vascular endothelial growth factor (VEGF), epidermal growth factor (EGF), and transforming growth factor beta (TGFb) growth factor families in the induction or persistence of fibrosis (Levitzki, Cytokine Growth Factor Rev, 2004, 15(4):229-35; Strutz et al., Kidney Intl, 2000, 57:1521-38; Strutz et al., 2003, Springer Semin Immunopathol, 24:459-76; Rice et al., 1999, Amer J Pathol, 155 (1) :213-221; Broekelmann et al., 1991, Proc Nat Acad Sci, 88:6642-6; Wynn, 2004, Nat Rev Immunol, 4(8):583-94).

PDGF, EGF and FGF family members are potent mitogens for mesenchymal cells such as smooth muscle cells, myofibroblasts and fibroblasts (Benito et al., 1993, Growth Regul 3 (3):172-9; Simm et al, 1998, Basic Res Cardiol, 93(S3):40-3; Klagsburn, Prog Growth Factor Res, 1989, 1(4):207-35; Kirkland et al., 1998, J Am Soc Nephrol, 9(8):1464-73), the very cells which supplant normal tissue in fibrosis and are believed to play a role in tissue remodeling (Abboud, 1995, Annu Rev Physiol., 57: 297-309; Jinnin et al., 2004, J Cell Physiol, online; Martinet et al., 1996, Arch Toxicol 18:127-39; Desmouliere, Cell Biology International, 1995, 19:471-6; Jelaska et al., Springer Semin Immunopathol, 2000, 21:385-95).

Inhibition of PDGF attenuates both liver fibrosis and lung fibrosis in experimental models, suggesting fibrosis in different organs may have a common origin (Borkham-Kamphorst et al., 2004, Biochem Biophys Res Commun; Rice et al., 1999, Amer J Pathol, 155 (1) :213-221). An ,EGF receptor kinase inhibitor was also active in this lung fibrosis model. Three-fold overexpression of an EGF family member, HB-EGF, in mouse pancreas islets was sufficient to cause development of fibrosis in both the exocrine and endocrine compartments (Means et al., 2003, Gastroenterology, 124 (4): 1020-36).

Similarly, FGF1/FGF2-deficient mice show dramatically decreased liver fibrosis after chronic carbon tetrachloride (CC14) exposure (Yu et al., 2003, Am J Pathol, 163 (4) :1653-62). FGF expression is increased in human renal interstitial fibrosis where it strongly correlates with interstitial scarring (Strutz et al., 2000, Kidney Intl, 57:1521-38) as well as in a model of experimental lung fibrosis (Barrios et al., 1997, Am J Physiol, 273 (2 Pt 1):L451-8), again lending credence to the idea that fibrosis in various tissues has a common basis.

In addition, elevated levels of VEGF have been observed in several studies in persons with asthma (Hoshino et al., 2001, J Allergy Clin Immunol 107:1034-39; Hoshino et al., 2001, J Allergy Clin Immunol 107:295-301; Kanazawa et al. 2002, Thorax 57:885-8; Asai et al., J Allergy Clin Immunol 110:571-5, 2002; Kanazawa et al., 2004, Am J Respir Crit Care Med, 169:1125-30). Inducible expression of VEGF in a transgenic mouse model induces an asthma-like phenotype, edema, angiogenesis and smooth muscle hyperplasia (Lee et al., 2004, Nature Med 10:1095-1103).

Finally, TGFb stimulates production of extracellular matrix proteins including fibronectin and collagens and is believed to play an important role in fibrosis in many tissues (Leask et al., 2004, FASEB J 18(7):816-27; Bartram et al., 2004, Chest 125 (2) :754-65; Strutz et al., 2003, Springer Semin Immunopathol, 24:459-76; Wynn, 2004, Nat Rev Immunol, 4(8):583-94). Inhibitors of TGFb production and signaling pathways are active in a number of fibrosis animal models (Wang et al., 2002, Exp Lung Res, 28:405-17; Laping, 2003, Curr Opin Pharmacol, 3(2):204-8).

As summarized above, several growth factors are upregulated in fibrosis and the inhibition of a single factor seems to reduce the severity of fibrosis in the fibrosis models.

### SUMMARY OF THE INVENTION

Surprisingly, we found that the compounds of above general formula I are effective in the treatment or prevention of specific fibrotic diseases.

The present invention thus relates to the use of the compounds of above general formula I for the preparation of a medicament for the treatment or prevention of specific fibrotic diseases.

The present invention also relates to a method for the treatment or prevention of specific fibrotic diseases, by administration to a patient in need thereof of a pharmaceutical composition comprising a compound of above general formula I, together with a pharmaceutically suitable carrier. The expression "patient" is meant to comprise the mammalian animal body, preferably the human body.

The present invention further relates to a pharmaceutical composition for the treatment or prevention of specific fibrotic diseases which comprises a compound of above general formula I alone or in combination with one or more further therapeutic agents.

### DETAILLED DESCRIPTION OF THE INVENTION

In accordance with the present invention, the compounds of above general formula I are the compounds 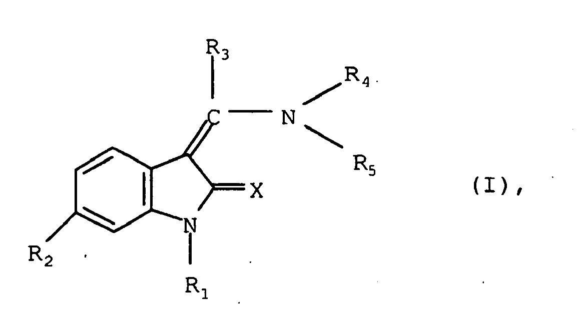 in which
X denotes an oxygen or sulphur atom,
R₁ denotes a hydrogen atom or a prodrug group such as a C₁₋₄-alkoxycarbonyl or C₂₋₄-alkanoyl group,
R₂ denotes a carboxy group, a straight-chain or branched C₁₋₆-alkoxycarbonyl group, a C₄₋₇-cycloalkoxycarbonyl or an aryloxycarbonyl group,
R₃ denotes a hydrogen atom, a C₁₋₆-alkyl, C₃₋₇-cycloalkyl, trifluoromethyl or heteroaryl group,
a phenyl or naphthyl group, or a phenyl or naphthyl group mono- or disubstituted by a fluorine, chlorine, bromine or iodine atom, by a trifluoromethyl, C₁₋₃-alkyl or C₁₋₃-alkoxy group, while in the event of disubstitution the substituents may be identical or different,
R₄ denotes a phenyl, pyrrolyl or furanyl group substituted by the group R₆, which may additionally be mono- or disubstituted by fluorine, chlorine, bromine or iodine atoms, by C₁₋₅-alkyl, trifluoromethyl, hydroxy, C₁₋₃-alkoxy, carboxy, C₁₋₃-alkoxycarbonyl, amino, acetylamino, C₁₋₃-alkyl-sulphonylamino, aminocarbonyl, C₁₋₃-alkyl-aminocarbonyl, di-(C₁₋₃-alkyl)-aminocarbonyl, aminosulphonyl, C₁₋₃-alkyl-aminosulphonyl, di-(C₁₋₃-alkyl)-aminosulphonyl, nitro or cyano groups, while the substituents may be identical or different and wherein
R₆ denotes an aminocarbonyl, C₁₋₄-alkylamino-carbonyl, N- (C₁₋₅-alkyl)-C₁₋₃-alkylaminocarbonyl, C₃₋₇-cycloalkyl-amino-carbonyl, N-(C₁₋₅-alkyl)-C₃₋₇-cycloalkylaminocarbonyl, (phenyl-C₁₋₃-alkyl ) amino-carbonyl, N-(C₁₋₃-alkyl)-phenyl-C₁₋₃-alkylamino-carbonyl group,
a C₁₋₃-alkylaminocarbonyl or N-(C₁₋₃-alkyl ) - C₁₋₃-alkylaminocarbonyl group wherein one or two alkyl moieties are substituted independently of one another by a nitro, cyano, carbamoyl, N-(C₁₋₃-alkyl)-carbamoyl, di-N-(C₁₋₃-alkyl)-carbamoyl, carboxy or C₁₋₃-alkoxycarbonyl group or are substituted in the 2- or 3-position by an amino, (C₁₋₃-alkyl)-amino, di-(C₁₋₃-alkyl)-amino, (C₁₋₄-alkoxycarbonyl) -amino, N- (C₁₋₄-alkoxycarbonyl) -N-(C₁₋₃-alkyl)-amino, piperazino, N- (C₁₋₃-alkyl)-piperazino, a 4- to 7-membered cycloalkyleneimino group, a hydroxy or methoxy group,
a 4- to 7-membered cycloalkyleneiminocarbonyl group wherein
   the cycloalkylene moiety may be fused to a phenyl ring via two adjacent ring atoms or may form a bridge to a methylene or ethylene group via two non-adjacent ring atoms or
   one or two hydrogen atoms may each be replaced by a C₁₋₃-alkyl group and/or
   in each case the methylene group in the 4 position of a 6- or 7-membered cycloalkyleneiminocarbonyl group may be substituted by a carboxy, C₁₋₄-alkoxycarbonyl, aminocarbonyl, C₁₋₃-alkylaminocarbonyl, di-(C₁₋₃-alkyl) -aminocarbonyl, di- (C₁₋₃-alkyl)-amino-C₁₋₃-alkyl, di- (C₁₋₃-alkyl)-amino, phenyl-C₁₋₃-alkylamino or N- (C₁₋₃-alkyl) - phenyl-C₁₋₃-alkylamino group, a hydroxy or methoxy group or
   may be replaced by an oxygen or sulphur atom, by a sulphinyl, sulphonyl or -NH group or by a nitrogen atom which is substituted by a C₁₋₃-alkyl, phenyl, C₁₋₃-alkyl-carbonyl, C₁₋₄-alkoxy-carbonyl, di-(C₁₋₃-alkyl)-amino-C₁₋₃ alkyl, ?-hydroxy-C₂₋₃-alkyl or benzoyl group,
   while all the single-bonded or fused phenyl groups contained in the groups mentioned under R₆ may be mono-or disubstituted by fluorine, chlorine, bromine or iodine atoms, by C₁₋₅-alkyl, trifluoromethyl, hydroxy, C₁₋₃-alkoxy, carboxy, C₁₋₃-alkoxycarbonyl, aminocarbonyl, C₁₋₄-alkylamino-carbonyl, di- (C₁₋₄-alkyl)-amino-carbonyl, aminosulphonyl, C₁₋₃-alkyl-aminosulphonyl, di- (C₁₋₃-alkyl) -aminosulphonyl, C₁₋₃-alkyl-sulphonylamino, nitro or cyano groups, while the substituents may be identical or different, or two adjacent hydrogen atoms of the phenyl groups may be replaced by a methylenedioxy group,
   and
R₅ denotes a hydrogen atom or a C₁₋₃-alkyl group,
   while by the term aryl group is meant a phenyl or naphthyl group optionally mono- or disubstituted by a fluorine, chlorine, bromine or iodine atom, by a cyano, trifluoromethyl, nitro, carboxy, aminocarbonyl, C₁₋₃-alkyl or C₁₋₃-alkoxy group and
   by the term heteroaryl group is meant a monocyclic 5- or 6-membered heteroaryl group optionally substituted in the carbon skeleton by a C₁₋₃-alkyl group, wherein
   the 6-membered heteroaryl group contains one, two or three nitrogen atoms and
   the 5-membered heteroaryl group contains an imino group optionally substituted by a C₁₋₃-alkyl or phenyl-C₁₋₃-alkyl group, an oxygen or sulphur atom or,
   an imino group optionally substituted by a C₁₋₃-alkyl or phenyl-C₁₋₃-alkyl group or an oxygen or sulphur atom and additionally a nitrogen atom or
   an imino group optionally substituted by a C₁₋₃-alkyl or phenyl-C₁₋₃-alkyl group and two nitrogen atoms,
   and moreover a phenyl ring may be fused to the abovementioned monocyclic heterocyclic groups via two adjacent carbon atoms and the bond is via a nitrogen atom or via a carbon atom of the heterocyclic moiety of a fused phenyl ring,
the hydrogen atoms in the abovementioned alkyl and alkoxy groups or in the alkyl moieties contained in the above-defined groups of formula I may be wholly or partly replaced by fluorine atoms,
the saturated alkyl and alkoxy moieties containing more than 2 carbon atoms present in the groups defined above, also include the branched isomers thereof such as for example the isopropyl, tert.butyl, isobutyl group, unless otherwise stated, and
wherein additionally the hydrogen atom of any carboxy group present or a hydrogen atom bound to a nitrogen atom, for example an amino, alkylamino or imino group or a saturated N-heterocycle such as the piperidinyl group, may be replaced in each case by a group which can be cleaved *in vivo,*
the tautomers, diastereomers, enantiomers and mixtures thereof and the salts thereof.

By a group which can be cleaved *in vivo* from an imino or amino group is meant, for example, a hydroxy group, an acyl group such as the benzoyl or pyridinoyl group or a C₁₋₁₆-alkanoyl group such as the formyl, acetyl, propionyl, butanoyl, pentanoyl or hexanoyl group, an allyloxycarbonyl group, a C₁₋₁₆-alkoxycarbonyl group such as the methoxycarbonyl, ethoxycarbonyl, propoxycarbonyl, isopropoxycarbonyl, butoxycarbonyl, tert.butoxycarbonyl, pentoxycarbonyl, hexyloxycarbonyl, octyloxycarbonyl, nonyloxycarbonyl, decyloxycarbonyl, undecyloxycarbonyl, dodecyloxycarbonyl or hexadecyloxycarbonyl group, a phenyl-C₁₋₆-alkoxycarbonyl group such as the benzyloxycarbonyl, phenylethoxycarbonyl or phenylpropoxycarbonyl groups, a C₁₋₃-alkylsulphonyl-C₂₋₄-alkoxycarbonyl, C₁₋₃-alkoxy-C₂₋₄-alkoxy-C₂₋₄-alkoxycarbonyl or RₑCO-O- (R_{f}CR_{g}) -O-CO group wherein
Rₑ denotes a C₁₋₈-alkyl, C₅₋₇-cycloalkyl, phenyl or phenyl-C₁₋₃-alkyl group,
R_{f} denotes a hydrogen atom, a C₁₋₃-alkyl, C₅₋₇-cycloalkyl or phenyl group and
Rg denotes a hydrogen atom, a C₁₋₃-alkyl or RₑCO-O- (R_{f}CR_{g})-O group wherein Rₑ to Rg are as hereinbefore defined,
wherein additionally the amino group may be a phthalimido group, while the ester groups mentioned above may also be used as a group which can be converted *in vivo* into a carboxy group.

An essential feature of the present invention is that R₆ denotes an unsubstituted aminocarbonyl group or an aminocarbonyl group which is substituted as defined hereinbefore or hereinafter.

Preferred compounds of general formula I are those wherein
X denotes an oxygen atom,
R₁ denotes a hydrogen atom, a C₁₋₄-alkoxycarbonyl or C₂₋₄-alkanoyl group,
R₂ denotes a carboxy group or a straight-chain or branched C₁₋₄-alkoxycarbonyl group,
R₃ denotes a hydrogen atom, a C₁₋₆-alkyl or C₃₋₇-cycloalkyl group,
a phenyl or naphthyl group, or a phenyl or naphthyl group mono- or disubstituted by a fluorine, chlorine or bromine atom, by a trifluoromethyl, C₁₋₃-alkyl or C₁₋₃-alkoxy group, while in the case of disubstitution the substituents may be identical or different,
R₄ denotes a furanyl group substituted by an aminocarbonyl, C₁₋₄-alkylaminocarbonyl or N-(C₁₋₄-alkyl)-C₁₋₃-alkylaminocarbonyl group, wherein the C₁₋₄-alkylaminocarbonyl or N-(C₁₋₄-alkyl)-C₁₋₃-alkylaminocarbonyl group may be substituted from position 2 in one or both alkyl moieties by an amino, C₁₋₃-alkylamino or di-(C₁₋₃-alkyl) amino group,
a pyrrolyl group substituted by an aminocarbonyl, C₁₋₄-alkylaminocarbonyl or N- (C₁₋₄-alkyl)-C₁₋₃-alkylaminocarbonyl group, wherein the C₁₋₄-alkylaminocarbonyl or N-(C₁₋₄-alkyl)-C₁₋₃-alkylaminocarbonyl group may be substituted from position 2 in one or both alkyl moieties by an amino, C₁₋₃-alkylamino or di- (C₁₋₃-alkyl) amino group and the nitrogen atom of the pyrrolyl ring is optionally substituted by a C₁₋₃-alkyl group, or
a phenyl group substituted by the group R₆, which may additionally be mono- or disubstituted by fluorine, chlorine or bromine atoms, by C₁₋₅-alkyl, trifluoromethyl, hydroxy, C₁₋₃-alkoxy, carboxy, C₁₋₃-alkoxycarbonyl, amino, acetylamino, C₁₋₃-alkyl-sulphonylamino, aminocarbonyl, C₁₋₃-alkyl-aminocarbonyl, di-(C₁₋₃-alkyl)-aminocarbonyl, aminosulphonyl, C₁₋₃-alkyl-aminosulphonyl, di- (C₁₋₃-alkyl) - aminosulphonyl, nitro or cyano groups, while the substituents may be identical or different and wherein
R₆ denotes an aminocarbonyl, C₁₋₄-alkylamino-carbonyl, N- (C₁₋₅-alkyl)-C₁₋₃-alkylaminocarbonyl, C₃₋₇-cycloalkyl-amino-carbonyl, N-(C₁₋₅-alkyl)-C₃₋₇-cycloalkylaminocarbonyl, (phenyl-C₁₋₃-alkyl) amino-carbonyl, N- (C₁₋₃-alkyl)-phenyl-C₁₋₃-alkylamino-carbonyl group,
a C₁₋₃-alkylaminocarbonyl or N-(C₁₋₃-alkyl)-C₁₋₃-alkylaminocarbonyl group wherein one or two alkyl moieties are substituted independently of one another by a nitro, cyano, carbamoyl, N- (C₁₋₃-alkyl) -carbamoyl, di-N-(C₁₋₃-alkyl)-carbamoyl, carboxy or C₁₋₃-alkoxycarbonyl group or are substituted in the 2- or 3-position by an amino, (C₁₋₃-alkyl) -amino, di- (C₁₋₃-alkyl)-amino,
(C₁₋₄-alkoxycarbonyl) -amino, N- (C₁₋₄-alkoxycarbonyl) -N-(C₁₋₃-alkyl)-amino, piperazino, N-(C₁₋₃-alkyl)-piperazino, a piperazinyl or piperidinyl group, a hydroxy or methoxy group,
a 4- to 7-membered cycloalkyleneiminocarbonyl group wherein the cycloalkylene moiety may be fused to a phenyl ring via two adjacent ring atoms or via two non-adjacent ring atoms may form a bridge to a methylene or ethylene group or
one or two hydrogen atoms may each be replaced by a C₁₋₃-alkyl group and/or
in each case the methylene group in the 4 position of a 6- or 7-membered cycloalkyleneiminocarbonyl group may be substituted by a carboxy, C₁₋₄-alkoxycarbonyl, aminocarbonyl, C₁₋₃-alkylaminocarbonyl, di-(C₁₋₃-alkyl)-aminocarbonyl, di-(C₁₋₃-alkyl)-amino-C₁₋₃-alkyl, di-(C₁₋₃-alkyl)-amino, phenyl-C₁₋₃-alkylamino or N-(C₁₋₃-alkyl) - phenyl-C₁₋₃-alkylamino group, a hydroxy or methoxy group or
may be replaced by an oxygen or sulphur atom, by a sulphinyl, sulphonyl or -NH group or by a nitrogen atom, which is substituted by a C₁₋₃-alkyl, phenyl, C₁₋₃-alkyl-carbonyl, C₁₋₄-alkoxy-carbonyl, di- (C₁₋₃-alkyl)-amino-C₁₋₃-alkyl, ?-hydroxy-C₂₋₃-alkyl or benzoyl group,
and
R₅ denotes a hydrogen atom or a C₁₋₃-alkyl group,
wherein the hydrogen atoms in the abovementioned alkyl and alkoxy groups or in the alkyl moieties contained in the above-defined groups of formula I may be wholly or partly replaced by fluorine atoms,
the saturated alkyl and alkoxy moieties containing more than 2 carbon atoms present in the groups defined above also include the branched isomers thereof such as for example the isopropyl, tert.butyl, isobutyl group, unless otherwise stated, and
additionally the hydrogen atom of any carboxy group present or a hydrogen atom bound to a nitrogen atom, for example an amino, alkylamino or imino group or a saturated N-heterocycle such as the piperidinyl group, may be replaced in each case by a group which can be cleaved *in vivo,*
the tautomers, diastereomers, enantiomers and mixtures thereof and the salts thereof.

A preferred sub-group relates to compounds of general formula I wherein
X denotes an oxygen atom,
R₁ denotes a hydrogen atom,
R₂ denotes a carboxy group or a C₁₋₂-alkoxycarbonyl group,
R₃ denotes a phenyl or naphthyl group, or a phenyl group monosubstituted by a fluorine, chlorine or bromine atom, by a trifluoromethyl, C₁₋₃-alkyl or C₁₋₃-alkoxy group,
R₄ denotes a pyrrolyl group substituted by an aminocarbonyl, C₁₋₄-alkylaminocarbonyl or N-(C₁₋₄-alkyl)-C₁₋₃-alkylaminocarbonyl group, wherein the C₁₋₄-alkylaminocarbonyl or N-(C₁₋₄-alkyl)-C₁₋₃-alkylaminocarbonyl group may be substituted from position 2 in one or both alkyl moieties by an amino, C₁₋₃-alkylamino or di- (C₁₋₃-alkyl) amino group and the nitrogen atom of the pyrrolyl ring is optionally substituted by a C₁₋₃-alkyl group, or
a phenyl group substituted in the 3- or 4-position by the group R₆, wherein
R₆ denotes an aminocarbonyl, C₁₋₄-alkylamino-carbonyl, N- (C₁₋₃alkyl) -C₁₋₃-alkylaminocarbonyl, C₅₋₆-cycloalkylamino-carbonyl, N- (C₁₋₅-alkyl)-C₅₋₆-cycloalkylaminocarbonyl group,
a C₁₋₃-alkylaminocarbonyl or N- (C₁₋₃-alkyl )-C₁₋₃-alkylaminocarbonyl group wherein one or two alkyl moieties are substituted independently of one another by a carbamoyl, N- (C₁₋₃-alkyl) -carbamoyl, di-N-(C₁₋₃-alkyl)-carbamoyl, C₁₋₃-alkoxycarbonyl group or are substituted in the 2- or 3-position by an amino, (C₁₋₃-alkyl) -amino, di-(C₁₋₃-alkyl)-amino, (C₁₋₄-alkoxycarbonyl) -amino, N-(C₁₋₄-alkoxycarbonyl)-N-(C₁₋₃-alkyl)-amino, piperazino, N-(C₁₋₃-alkyl)-piperazino, a piperazinyl or piperidinyl group, a hydroxy or methoxy group,
a piperidinocarbonyl, piperazinocarbonyl, homopiperazinocarbonyl or 2,3,4,5-tetrahydro-1(H)-azepinocarbonyl group,
which may be fused to a phenyl ring via two adjacent unsubstituted carbon atoms or
may be substituted in the 4 position by a C₁₋₃-alkyl, C₁₋₄-alkoxycarbonyl, di- (C₁₋₃-alkyl) -amino, di- (C₁₋₃-alkyl)-amino-C₁₋₃-alkyl, 2-hydroxy-ethyl, hydroxy or methoxy group,
or a 2,5-diaza-bicyclo[2.2.1]hept-2-yl-carbonyl group which may be substituted in the 5 position by a C₁₋₃-alkyl group,
and
R₅ denotes a hydrogen atom or a C₁₋₃-alkyl group,
wherein the hydrogen atoms in the abovementioned methyl and methoxy groups may be replaced by 1, 2 or 3 fluorine atoms, and
the saturated alkyl and alkoxy moieties containing more than 2 carbon atoms which are present in the groups defined above also include the branched isomers thereof, such as, for example, the isopropyl, tert.butyl and isobutyl group,
the tautomers, diastereomers, enantiomers and mixtures thereof and the salts thereof.

Particularly preferred compounds of general formula I are those wherein
X denotes an oxygen atom,
R₁ and R₅ each denote a hydrogen atom,
R₂ denotes a methoxycarbonyl group,
R₃ denotes a phenyl group and
R₄ denotes a phenyl group which is monosubstituted in the 3- or 4-position by the group R₅, wherein
R₆ denotes an aminocarbonyl, C₁₋₃-alkylamino-carbonyl, N- (C₁₋₅-alkyl)-C₁₋₃-alkylaminocarbonyl, cyclohexylaminocarbonyl, N- (C₁₋₅-alkyl) - cyclohexylaminocarbonyl, phenyl-C₁₋₃-alkylamino-carbonyl, N- (C₁₋₃-alkyl)-phenyl-C₁₋₃-alkylamino-carbonyl,
a piperidinocarbonyl, 4-hydroxy-piperidinocarbonyl, 4-[di-(C₁₋₃-alkyl)-amino]-piperidinocarbonyl, 4-[di-(C₁₋₃-alkyl)-amino-C₁₋₃-alkyl]-piperidinocarbonyl, piperazinocarbonyl, N-(C₁₋₃-alkyl)-piperazinocarbonyl, N-(C₁₋₄-alkoxycarbonyl)-piperazinocarbonyl, N- [di-(C₁₋₃-alkyl) -amino-C₁₋₃-alkyl]-piperazinocarbonyl, N-(2-hydroxy-ethyl)-piperazinocarbonyl, homopiperazinocarbonyl, N-(C₁₋₃-alkyl)-homopiperazinocarbonyl, 2,3,4,5-tetrahydro-1(H)-benzo[d]azepinocarbonyl or 5-methyl-2,5-diaza-bicyclo[2.2.1]hept-2-yl-carbonyl group,
a C₁₋₃-alkylaminocarbonyl or N-(C₁₋₅-alkyl)-C₁₋₃-alkylaminocarbonyl group wherein one or two alkyl moieties are substituted by a carbamoyl group or are substituted in the 2- or 3-position by an amino, (C₁₋₃-alkyl) -amino, di-(C₁₋₃-alkyl)-amino, hydroxy or methoxy group,
the tautomers, diastereomers, enantiomers and mixtures thereof and the salts thereof.

The following are mentioned as examples of most particularly preferred compounds:
(a) methyl 3-(Z)-[1-{4-[N-(2-dimethylamino-ethyl)-N-methylcarbamoyl]-phenylamino}-1-phenyl-methylidene]-2-indolinone-6-carboxylate
(b) methyl 3-(Z)-[1-{4-[N-(3-dimethylamino-propyl)-N-methyl-carbamoyl]-phenylamino}-1-phenyl-methylidene]-2-indolinone-6-carboxylate
(c) methyl 3-(Z)-[1-{4-[(4-methyl-piperazin-1-yl)-carbonyl]-phenylamino}-1-phenyl-methylidene]-2-indolinone-6-carboxylate
(d) methyl 3-(Z)-[1-{4-[(4-hydroxy-piperidin-1-yl)-carbonyl]-phenylamino}-1-phenyl-methylidene]-2-indolinone-6-carboxylate
(e) methyl 3-(Z)-[1-{4-[(piperazin-1-yl)-carbonyl]-phenylamino}-1-phenyl-methylidene]-2-indolinone-6-carboxylate
(f) methyl 3-(Z)-[1-{4-[N-(2-methylamino-ethyl)-N-methylcarbamoyl]-phenylamino}-1-phenyl-methylidene]-2-indolinone-6-carboxylate
(g) methyl 3-(Z)-[1-{4-[(4-dimethylamino-piperidin-1-yl)-carbonyl]-phenylamino}-1-phenyl-methylidene]-2-indolinone-6-carboxylate
(h) methyl 3-(Z)-[1-{4-[(4-ethyl-piperazin-1-yl)-carbonyl]-phenylamino}-1-phenyl-methylidene]-2-indolinone-6-carboxylate
(i) methyl 3-(Z)-[1-{4-[(4-(2-hydroxy-ethyl)-piperazin-1-yl)-carbonyl]-phenylamino}-1-phenyl-methylidene]-2-indolinone-6-carboxylate
(k) methyl 3-(Z)-{1-[4-(5-methyl-2,5-diaza-bicyclo[2.2.1]hept-2-yl-carbonyl)-phenylamino]-1-phenyl-methylidene}-2-indolinone-6-carboxylate,
   their tautomers, their stereoisomers or the physiologically acceptable salts thereof.

The above exemplified compounds, their tautomers, their stereoisomers or the physiologically acceptable salts thereof, as well as their manufacturing process, have been described in WO 02/81445, the content of which is incorporated herein by reference.

Further compounds in accordance with the above general formula I which are preferred within the meaning of the present invention are the following compounds:

### (1) (S)-3-(Z)-[1-{4-[(3,4-dimethyl-piperazin-1-yl)-carbonyl]-phenylamino}-1-phenyl-methylidene] -6-methoxycarbonyl-2-indolinone

Prepared from 1-acetyl-3-(1-ethoxy-1-phenylmethylene)-6-methoxycarbonyl-2-indolinone and *(S)*-4-(3,4-dimethylpiperazin-1-yl-carbonyl)-aniline.
Melting point: 265-266 °C
C₃₀H₃₀N₄O₄
Mass spectrum: m/z = 511 [M+H]⁺

(m) 3-(Z)-[1-{5-[(2-dimethylamino-ethyl)-N-methylcarbamoyl]-furan-2-yl-amino}-1-phenyl-methylidene]-6-methoxycarbonyl-2-indolinone
Prepared from 1-acetyl-3-(1-ethoxy-1-phenylmethylene)-6-methoxycarbonyl-2-indolinone and 2-amino-5-[(2-dimethylamino-ethyl)-N-methyl-carbamoyl]-furan. R_{f}-value: 0,30 (silica gel, methylene chloride/methanol = 9:1)
C₂₇H₂₈N₄O₅
Mass spectrum: m/z = 489 [M+H]⁺
their tautomers, their stereoisomers or the physiologically acceptable salts thereof.

These compounds may be prepared analogously to the compounds of WO 02/81445 and using the methods described therein.

Tautomers, stereoisomers or physiologically acceptable salts of these compounds are also contemplated within the scope of the present invention, and may be obtained using the methods described in WO 02/81445, the content of which is herein incorporated by reference.

The following list of specific compounds is illustrative of the present invention, without constituting any limitation of its scope:
(1) methyl 3-(Z)-[1-{4-[*N*-(2-dimethylamino-ethyl)-*N-*methyl-carbamoyl]-phenylamino}-1-phenyl-methylidene]-2-indolinone-6-carboxylate
(2) methyl 3-(Z)-[1-{4-[(2-dimethylamino-ethyl)-carbamoyl]-phenylamino}-1-phenyl-methylidene]-2-indolinone-6-carboxylate
(3) methyl 3-(Z)-[1-{4-[(3-dimethylamino-propyl)-carbamoyl]-phenylamino}-1-phenyl-methylidene]-2-indolinone-6-carboxylate
(4) methyl 3-(Z)-[1-{4-[*N*-(3-dimethylamino-propyl)-*N-*methyl-carbamoyl]-phenylamino}-1-phenyl-methylidene]-2-indolinone-6-carboxylate
(5) methyl 3-(Z)-[1-{4-[(2-dimethylamino-ethyl)-*N*-ethylcarbamoyl]-phenylamino}-1-phenyl-methylidene]-2-indolinone-6-carboxylate
(6) methyl 3-(Z)-[1-{4-[(2-(tert-butyloxycarbonyl-N-methylamino)-ethyl)-N-methyl-carbamoyl]-phenylamino}-1-phenyl-methylidene]-2-indolinone-6-carboxylate
(7) methyl 3-(Z)-[1-{4-[N,N-bis-(2-diethylamino-ethyl)-carbamoyl]-phenylamino}-1-phenyl-methylidene]-2-indolinone-6-carboxylate
(8) methyl 3-(Z)-[1-{3-[(2-dimethylamino-ethyl)-carbamoyl]-phenylamino}-1-phenyl-methylidene]-2-indolinone-6-carboxylate
(9) methyl 3-(Z)-[1-{3-[(2-dimethylamino-ethyl)-N-methylcarbamoyl]-phenylamino}-1-phenyl-methylidene]-2-indolinone-6-carboxylate
(10) methyl 3-(Z)-[1-{3-[(3-dimethylamino-propyl)-carbamoyl]-phenylamino}-1-phenyl-methylidene]-2-indolinone-6-carboxylate
(11) methyl 3-(Z)-[1-{3-[(3-dimethylamino-propyl)-N-methylcarbamoyl]-phenylamino}-1-phenyl-methylidene]-2-indolinone-6-carboxylate
(12) methyl 3-(Z)-[1-{4-[(4-methyl-piperazin-1-yl)-carbonyl]-phenylamino}-1-phenyl-methylidene]-2-indolinone-6-carboxylate
(13) methyl 3-(Z)-[1-{4-[(piperidin-1-yl)-carbonyl]-phenylamino}-1-phenyl-methylidene]-2-indolinone-6-carboxylate
(14) methyl 3-(Z)-[1-{4-[N-cyclohexyl-N-methyl-carbamoyl]-phenylamino}-1-phenyl-methylidene]-2-indolinone-6-carboxylate
(15) methyl 3-(Z)-[1-{4-[isopropyl-carbamoyl]-phenylamino}-1-phenyl-methylidene]-2-indolinone-6-carboxylate
(16) methyl 3-(Z)-[1-{4-[2,3,4,5-tetrahydro-1(H)-benzo[d]azepin-3-yl-carbonyl]-phenylamino}-1-phenyl-methylidene]-2-indolinone-6-carboxylate
(17) methyl 3-(Z)-[1-{4-[(4-hydroxy-piperidin-1-yl)-carbonyl]-phenylamino}-1-phenyl-methylidene]-2-indolinone-6-carboxylate
(18) methyl 3-(Z)-[1-{4-[(4-tert-butyloxycarbonylpiperazin-1-yl)-carbonyl]-phenylamino}-1-phenyl-methylidene]-2-indolinone-6-carboxylate
(19) methyl 3-(Z)-[1-{4-[(4-tert-butyloxycarbonyl-[1,4]diazepan-1-yl)-carbonyl]-phenylamino}-1-phenyl-methylidene]-2-indolirione-6-carboxylate
(20) methyl 3-(Z)-[1-(4-carbamoyl-phenylamino)-1-phenyl-methylidene]-2-indolinone-6-carboxylate
(21) methyl 3-(Z)-[1-(4-propylcarbamoyl-phenylamino)-1-phenyl-methylidene]-2-indolinone-6-carboxylate
(22) methyl 3-(Z)-[1-(4-dimethylcarbamoyl]-phenylamino)-1-phenyl-methylidene]-2-indolinone-6-carboxylate
(23) methyl 3-(Z)-[1-{3-[N-(carbamoyl-methyl)-N-methylcarbamoyl]-phenylamino}-1-phenyl-methylidene]-2-indolinone-6-carboxylate
(24) methyl 3-(Z)-[1-{3-[N-(2-methoxy-ethyl)-N-methylcarbamoyl]-phenylamino}-1-phenyl-methylidene]-2-indolinone-6-carboxylate
(25) methyl 3-(Z)-[1-{3-[(2-carbamoyl-ethyl)-carbamoyl] - phenylamino}-1-phenyl-methylidene]-2-indolinone-6-carboxylate
(26) methyl 3-(Z)-[1-{3-[N,N-bis-(2-hydroxy-ethyl)-carbamoyl]-phenylamino}-1-phenyl-methylidene]-2-indolinone-6-carboxylate
(27) methyl 3-(Z)-[1-{1-methyl-2-[(2-dimethylamino-ethyl)-N-methyl-carbamoyl]-pyrrol-4-yl-amino}-1-phenyl-methylidene]-2-indolinone-6-carboxylate
(28) methyl 3-(Z)-[1-{4-[(4-dimethylamino-piperidin-1-yl)-carbonyl]-phenylamino}-1-phenyl-methylidene]-2-indolinone-6-carboxylate
(29) methyl 3-(Z)-[1-{4-[(4-ethyl-piperazin-1-yl)-carbonyl]-phenylamino}-1-phenyl-methylidene]-2-indolinone-6-carboxylate
(30) methyl 3-(Z)-[1-{4-[(4-(2-dimethylamino-ethyl)-piperazin-1-yl)-carbonyl]-phenylamino}-1-phenyl-methylidene]-2-indolinone-6-carboxylate
(31) methyl 3-(Z)-[1-{4-[(4-(2-hydroxy-ethyl)-piperazin-1-yl)-carbonyl]-phenylamino}-1-phenyl-methylidene]-2-indolinone-6-carboxylate
(32) methyl 3-(Z)-{1-[4-(5-methyl-2,5-diazabicyclo[2.2.1]hept-2-yl-carbonyl)-phenylamino]-1-phenyl-methylidene}-2-indolinone-6-carboxylate
(33) methyl 3-(Z)-[1-{4-[(4-tert-butyloxycarbonyl-trans-2,5-dimethyl-piperazin-1-yl)-carbonyl]-phenylamino}-1-phenyl-methylidene]-2-indolinone-6-carboxylate
(34) methyl 3-(Z)-[1-{4-[(4-dimethylaminomethyl-piperidin-1-yl)-carbonyl]-phenylamino}-1-phenyl-methylidene]-2-indolinone-6-carboxylate
(35) methyl 3-(Z)-[1-{4-[(cis-3,5-dimethyl-piperazin-1-yl)-carbonyl]-phenylamino}-1-phenyl-methylidene]-2-indolinone-6-carboxylate
(36) methyl (*R*)-3- (z) -[1-{4-[(3,4-dimethyl-piperazin-1-yl) - carbonyl]-phenylamino}-1-phenyl-methylidene]-2-indolinone-6-carboxylate
(37) methyl 3-(Z)-[1-{4-[(4-(2-diethylamino-ethoxy)-piperidin-1-yl)-carbonyl]-phenylamino}-1-phenyl-methylidene]-2-indolinone-6-carboxylate
(38) methyl 3-(Z)-[1-{4-[(3-(2-diethylamino-ethoxy)-pyrrolidin-1-yl)-carbonyl]-phenylamino}-1-phenyl-methylidene]-2-indolinone-6-carboxylate
(39) methyl 3-(Z)-[1-{4-[(3-dimethylamino-pyrrolidin-1-yl)-carbonyl]-phenylamino}-1-phenyl-methylidene]-2-indolinone-6-carboxylate
(40) methyl 3-(Z)-[1-{4-[(piperazin-1-yl)-carbonyl]-phenylamino}-1-phenyl-methylidene]-2-indolinone-6-carboxylate -trifluoroacetate
(41) methyl 3-(Z)-[1-{4-[([1,4]diazepan-1-yl)-carbonyl]-phenylamino}-1-phenyl-methylidene]-2-indolinone-6-carboxylate -trifluoroacetate
(42) methyl 3-(Z)-[1-{4-[*N*-(2-methylamino)-ethyl)-*N*-methylcarbamoyl]-phenylamino}-1-phenyl-methylidene]-2-indolinone-6-carboxylate
(43) methyl 3-(Z)-[1-{4-[(trans-2,5-dimethyl-piperazin-1-yl)-carbonyl]-phenylamino}-1-phenyl-methylidene]-2-indolinone-6-carboxylate -trifluoroacetate
(44) 3-(Z)-[1-{4-[(4-methyl-piperazin-1-yl)-carbonyl]-phenylamino}-1-phenyl-methylidene]-2-indolinone-6-carboxylic acid
(45) methyl 3-(Z)-[1-{4-[N,N-bis-(2-dimethylamino-ethyl)-carbamoyl]-phenylamino}-1-phenyl-methylidene]-2-indolinone-6-carboxylate
(46) methyl 3-(Z)-[1-{4-[N,N-bis-(3-diethylamino-propyl)-carbamoyl]-phenylamino}-1-phenyl-methylidene]-2-indolinone-6-carboxylate
(47) methyl 3-(Z)-[1-{4-[(2-diethylamino-ethyl)-carbamoyl]-phenylamino}-1-phenyl-methylidene]-2-indolinone-6-carboxylate
(48) methyl 3-(Z)-[1-{4-[N,N-bis-(2-hydroxy-ethyl)-carbamoyl]-phenylamino}-1-phenyl-methylidene]-2-indolinone-6-carboxylate
(49) methyl 3-(Z)-[1-{4-[N-(carbambol-methyl)-N-methylcarbamoyl]-phenylamino}-1-phenyl-methylidene]-2-indolinone-6-carboxylate
(50) methyl 3-(Z)-[1-{4-[(2-carbamoyl-ethyl)-carbamoyl]-phenylamino}-1-phenyl-methylidene]-2-indolinone-6-carboxylate
(51) methyl 3-(Z)-[1-{3-[N-(2-hydroxy-ethyl)-N-methylcarbamoyl]-phenylamino}-1-phenyl-methylidene]-2-indolinone-6-carboxylate
(52) methyl 3-(Z)-[1-{3-[N-(2-methylamino-ethyl)-N-methylcarbamoyl]-phenylamino}-1-phenyl-methylidene]-2-indolinone-6-carboxylate
(53) methyl 3-(Z)-[1-{3-[N-(2-aminoethyl)-carbamoyl]-phenylamino}-1-phenyl-methylidene]-2-indolinone-6-carboxylate
(54) methyl 3-(Z)-[1-{4-[(2-(tert-butyloxycarbonyl-amino)-ethyl)-carbamoyl]-phenylamino}-1-phenyl-methylidene]-2-indolinone-6-carboxylate
(55) methyl 3-(Z)-[1-{5-[(2-dimethylamino-ethyl)-N-methylcarbamoyl]-furan-2-yl-amino}-1-phenyl-methylidene]-2-indolinone-6-carboxylate
(56) methyl 3-(Z)-[1-{4-[(2-amino-ethyl)-carbamoyl]-phenylamino}-1-phenyl-methylidene]-2-indolinone-6-carboxylate
(57) methyl 3-(Z)-[1-{4-[(4-(2-dimethylamino-ethoxy)-piperidin-1-yl)-carbonyl]-phenylamino}-1-phenyl-methylidene]-2-indolinone-6-carboxylate
(58) methyl 3-(Z)-[1-{4-[(3-(2-dimethylamino-ethoxy)-pyrrolidin-1-yl)-carbonyl]-phenylamino}-1-phenyl-methylidene]-2-indolinone-6-carboxylate
(59) methyl *(S)*-3-(Z)-[1-{4-[(3,4-dimethyl-piperazin-1-yl)-carbonyl]-phenylamino}-2-phenyl-methylidene]-2-indolinone-6-carboxylate
(60) (*S*)-3-(Z)-[1-{4-[(3,4-dimethyl-piperazin-1-yl)-carbonyl]-phenylamino}-1-phenyl-methylidene]-6-methoxycarbonyl-2-indolinone
(61) 3-(Z)-[1-{5-[(2-dimethylamino-ethyl)-N-methylcarbamoyl]-furan-2-yl-amino}-1-phenyl-methylidene]-6-methoxycarbonyl-2-indolinone
as well as their tautomers, their stereoisomers or the physiologically acceptable salts thereof.

The compounds of general formula I, their tautomers, their stereoisomers or the physiologically acceptable salts thereof are thus suitable for the prevention or treatment of a specific fibrotic disease selected from the group consisting of:
Fibrosis and remodeling of lung tissue in chronic obstructive pulmonary disease (COPD), chronic bronchitis, and emphysema;
Lung fibrosis and pulmonary diseases with a fibrotic component including but not limited to idiopathic pulmonary fibrosis (IPF), giant cell interstitial pneumonia (GIP), sarcodosis, cystic fibrosis, respiratory distress syndrome (ARDS), granulomatosis, silicosis, drug-induced lung fibrosis (for example, induced by drugs such as bleomycin, bis-chloronitrosourea, cyclophosphamide, amiodarone, procainamide, penicillamine, gold or nitrofurantoin), silicosis, asbestosis, systemic scleroderma;
Fibrosis and remodeling in asthma;

In a preferred embodiment in accordance with the present invention, the compounds of general formula I, their tautomers, their stereoisomers or the physiologically acceptable salts thereof are especially suitable for the prevention or treatment of idiopathic pulmonary fibrosis.

### BIOLOGICAL ACTIVITY

The following experimental results illustrate the present invention without representing a limitation of its scope.

### Abreviations

DEPC (diethylpyrocarbonate)
dNTP (deoxyribonucleotide triphosphates)
CT (Cycle at which amplification reaches a set Threshold)
DNA (deoxyribonucleic acid)
cDNA (complementary DNA)
RNA (ribonucleic acid)
mRNA (messenger RNA)
PCR (polymerase chain reaction)

### Example B1:

In the following experiments of Example B1, Example A denotes the compound methyl 3-(Z)-[1-{4-[(4-methyl-piperazin-1-yl)-carbonyl]-phenylamino}-1-phenyl-methylidene]-2-indolinone-6-carboxylate, which is compound (12) of the list of compounds and compound (c) of the preferred list of compounds.

(A) Effect of a representative compound on lung morphology following bleomycin-induced pulmonary fibrosis.

### Materials and Methods

Bleomycin sulfate (Bleomycin HEXAL^{™}) was purchased from a local pharmacy.

### Bleomycin administration and treatment protocols

All experiments were performed in accordance with German guidelines for animal welfare, performed by persons certified to work with animals and approved by the responsible authorities. Male Wistar rats were intratracheally injected with Bleomycin sulfate (10U/kg body weight in 300µl saline) or saline alone (saline control) using a catheter (0,5mm internal diameter, 1.0mm external diameter) through the nasal passage, following exposure to the anaesthetic Isofluorane for 5 minutes. The following day, the rats were orally treated with Example A (compound (12)) or saline suspended in 1ml 0.1% Natrosol. Control rats were administered 1ml 0.1% Natrosol (vehicle control).

A total of 30 rats were investigated and were grouped and treated as shown in Table 1.

**Table 1**

| Intratracheal instillation | No. of animals | Compound | Treatment Schedule |
|---|---|---|---|
| | | | |
| Bleomycin 10U /kg | 10 | Example A (Compound (12) ) | Days 1-21 |
| Bleomycin 10U /kg | 10 | Vehicle only | Days 1-21 |
| Saline (300µl) | 10 | Vehicle only | Days 1-21 |

21 days following bleomycin instillation, the rats were killed with a lethal intraperitoneal injection of Narcoren^{™} (Pentobarbital Sodium, Rhone Merieux). The lungs were then removed, blotted dry and half was snap frozen in liquid nitrogen and stored at -80°C. The other half was fixed in 4% formalin for subsequent paraffin embedding and histology.

### Histology

The lung tissues fixed in 4% formalin were embedded into paraffin and 5µm sections were cut using a microtome (Leica SM200R) and placed on poly-L-lysine coated slides. The sections were then dried onto the slides (60°C 2 hours) and then left to cool at room temperature. Collagen deposition was assessed using Masson's Trichrome staining.

### Results

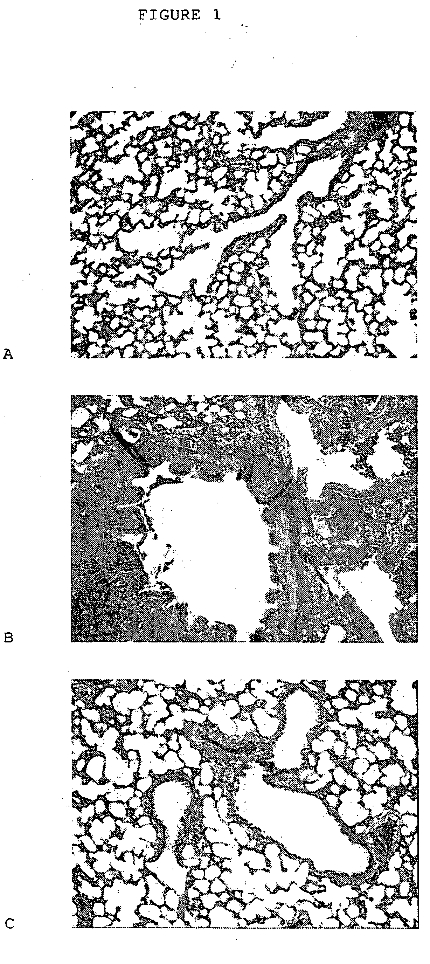

Figure 1A shows the result obtained with the control group, which received saline and the vehicle instead of bleomycin intratracheally.

Rats treated intratracheally with bleomycin and the vehicle developed severe lung fibrosis, as seen in Figure 1B. The alveoli have been largely replaced by fibroblasts and extracellular matrix and the normal lung structure is nearly obliterated.

Daily treatment of bleomycin-treated rats with 50 mg/kg of Example A (compound (12)) showed a consistent, nearly complete reversal of lung fibrosis in this model. A typical example is shown in Figure 1C. Alveoli are intact and little or no fibroblast infiltration or extracellular matrix deposition has occurred. Normal lung structure has been maintained, which is evidenced by a comparison of Figure 1C with Figure 1A.

(B) Effect of a representative compound on expression of fibrotic marker genes following bleomycin-induced pulmonary fibrosis.

### mRNA extractions and synthesis of cDNA

One part of the frozen lung tissue dedicated to investigation of gene expression was cut into small pieces using a sterile scalpel blade. Approximately 100mg of tissue was then placed into a 2ml Eppendorf tube and 1.5ml of Trizol (Invitrogen) was added. A sterile tungsten carbide bead (Qiagen) was then added to the tube and the tube was placed in a Retsch MM300 Tissue disruptor (Qiagen) at a frequency of 30.0Hz for 8 minutes. After this time, the bead was removed and the sample centrifuged at 12000rpm for 10 minutes to remove tissue debris. The RNA was extracted using a modified version of the manufacturer's protocol supplied with Trizol. Briefly, 0.3ml chloroform was added to the tube and the tube shaken vigorously and then left to incubate at room temperature for 5 minutes, after which the tube was centrifuged for 15 minutes at 12000 rpm at 4°C. The upper colorless aqueous phase was then collected and added to 750µl isopropanol. This was then shaken vigorously and stored at -80°C overnight. The samples were then incubated at room temperature for 15 minutes, after which they were centrifuged for 40 minutes at 12000 rpm at 4°C. The supernatant was then removed and 500µl of 70% ethanol was added to wash the pellet then the sample was centrifuged for 10 minutes at 12000 rpm an 4°C, this wash step was repeated twice, after which the pellet was left to dry for 10 - 15 minutes. Finally the pellet was resuspended in µl RNase free water and stored at -80°C. The concentration of each sample was then measured using a spectrophotometer.

Using the Superscript^{™} III (Invitrogen, Paisley, UK) RT-first strand synthesis kit, 2µg of each mRNA sample was reversed transcribed using a modified version of the manufacturer's protocol. Briefly, a mixture of 2µg RNA, 1µl random hexamer primers (50ng/µl), 1µl dNTP mix (10mM) was made up to 10µl with DEPC-treated water and incubated at 65°C for 5 minutes, after which it was placed on ice for 5 minutes. Following this, to each reaction, 2µl RT buffer (10X), 4µl MgCl₂ (25mM), 2µl DTT (0.1M), 1µl RNaseOUT^{™} (40U/µl) and 1µl SuperScript™ III enzyme (200U/µl) was added and the mixture placed in a thermal cycler (Applied Biosystems) under the following conditions: 25° C for 10 minutes, 50°C for 50 minutes and 85°C for 5 minutes, after which 1µl of RNase H was added and incubated at 37°C for 20 minutes. The synthesized cDNA was diluted to 5ng/µl using the assumption that the RT reaction fully transcribed all of the mRNA to cDNA and was a concentration of 100ng/µl.

### Investigation of gene expression using real time PCR

Gene expression was investigated in each of the samples using the Applied Biosystems 7700 sequence detection system. Primers for the 18S endogenous control were purchased as pre-developed assay reagent kits, whereas primers and probes (see Table 2 below) for pro-collagen I and fibronectin were designed using PrimerExpress™ (Applied Biosystems), ensuring that at least one of the primers or probes in each set overlapped an intron / exon junction, thus eliminating the possibility of amplifying any contaminating genomic DNA in the cDNA sample. The purchased PDARs also amplified only cDNA.

**Table 2**

| Target | | Sequence |
|---|---|---|
| | | |
| Fibronectin | Forward | 5'-GAT GCC GAT CAG AAG TTT GGA-3' |
| | Reverse | 5' -TCG TTG GTC GTG CAG ATC TC-3' |
| | Probe | 5′-FAM-CTG CCC AAT GGC TGC CCA TGA- TAMRA-3′ |
| | | |
| Pro-Collagen I | Forward | 5'-CAG ACT GGC AAC CTG AAG AAG TC- |
| | Reverse | 5'-TCG CCC CTG' AGC TCG AT-3' |
| | Probe | 5'-FAM-CTG CTC CTC CAG GGC TCC AAC GA-TAMRA3' |

Real Time PCR was carried out in 25µl reactions, using 25ng (5µl) of cDNA per reaction. A quantitative PCR core kit was purchased (Eurogentec) and a master-mix was made up as follows for 100 reactions: 500µl 10X reaction buffer, 500µl MgCl₂ (50mM), 200µl dNTP mix solution (5mM), 25µl Hot Goldstar enzyme, 75µl 18S PDAR, 22.5µl forward primer, 22.5µl reverse primer, 15µl probe and 640µl DEPC treated water. 20µl of this master-mix was then added to 25ng (5µl) target cDNA. Each analysis was carried out in triplicate.

In order to quantify the gene expression, a standard curve was constructed for each primer set and was included on each plate. The standards were made up of a mix of all the cDNA's under investigation; this mix of cDNA's was serially diluted 10, 20, 50, 100, 100 times. A standard curve was constructed of the obtained C_{T} (Cycle at which amplification reaches a set Threshold) against the LOG₁₀ of the dilution factor. Curves were drawn for the target gene and the 18S rRNA endogenous control. The C_{T} value for both targets for each of the samples was then converted to a fold dilution using the standard curve and the target gene value was normalized to the 18S gene value.

### Statistics

All statistical analyses were carried out using GraphPad Prism V 4.02 software. Comparisons were made using a non-parametric T-test (Mann-Whitney U test) and a significant value was considered to be p = 0.05.

### Results

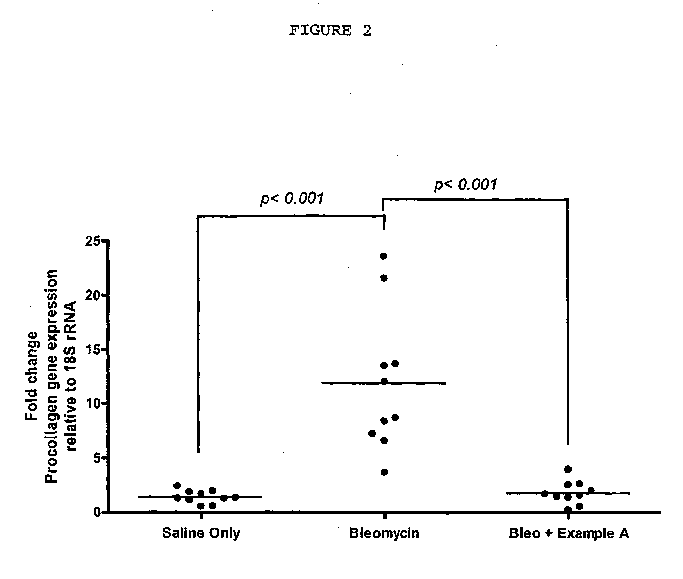

The results are shown in Figures 2 (procollagen I) and 3 (fibronectin). Each data point represents RNA isolated from the lung of a single rat.

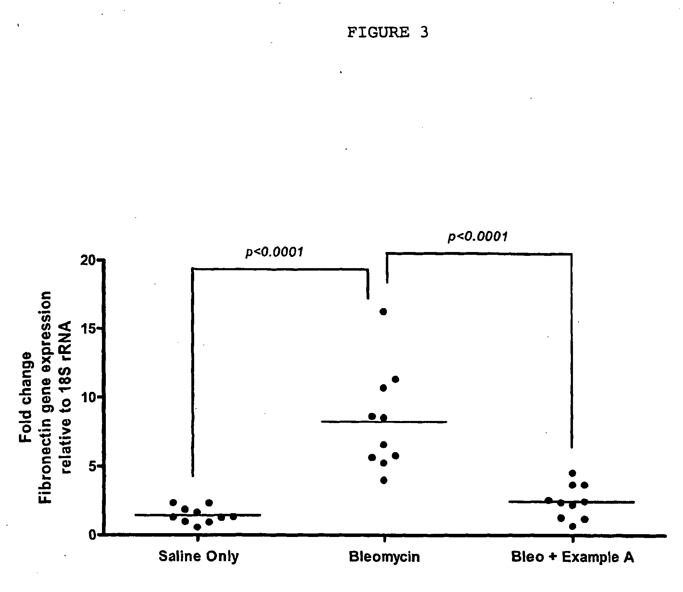

Intratracheal administration of bleomycin and subsequent treatment with vehicle only showed large increases in procollagen I and fibronectin gene expression in the lung, as seen in Figures 2 and 3, consistent with the histologically apparent lung fibrosis seen in Figure 1B.

Daily treatment of Bleomycin-treated rats with 50 mg/kg of Example A (compound (12)) showed a significant (p ≤ 0.0001) inhibition of expression of fibrotic marker genes in this model, as seen in Figures 2 and 3.

This experiment thus demonstrates that expression of fibrotic markers, and therefore deposition of extracellular matrix, may be dramatically reduced by treatment with Example A (compound (12)).

Thus, expression of fibrotic markers, and therefore deposition of extracellular matrix, may be dramatically reduced by treatment with the compounds in accordance with the present invention.

By reason of their biological properties the compounds according to the invention may be used in monotherapy or in conjunction with other pharmacologically active compounds. Such pharmacologically active compounds may be compounds which are, for example, also pharmacologically active in the treatment of fibrosis. Such pharmacologically active compounds may also be substances with a secretolytic, broncholytic and/or anti-inflammatory activity.

In a preferred embodiment in accordance with the present invention, such pharmacologically active compounds are preferably selected from the group consisting of anticholinergic agents, beta-2 mimetics, steroids, PDE-IV inhibitors, p38 MAP kinase inhibitors, NK₁ antagonists, LTD4 antagonists, EGFR inhibitors and endothelin-antagonists.

Anticholinergic agents may preferably be selected from the group consisting of the tiotropium salts, oxitropium salts, flutropium salts, ipratropium salts, glycopyrronium salts and trospium salts.

Beta-2 mimetics may preferably be selected from the beta-2 mimetics disclosed, for example, in US 4,460,581, which is incorporated herein by reference.

PDE-IV inhibitors may preferably be selected from the group consisting of enprofyllin, theophyllin, roflumilast, ariflo (cilomilast), CP-325,366, BY343, D-4396 (Sch-351591), AWD-12-281 (GW-842470), N-(3,5-dichloro-1-oxo-pyridin-4-yl)-4-difluoromethoxy-3-cyclopropylmethoxybenzamide, NCS-613, pumafentine, (-) p- [ (4*a*R*,10*b*S*) - 9-ethoxy-1, 2, 3, 4, 4a, 10b-hexahydro-8-methoxy-2-methylbenzo[s][1,6]naphthyridin-6-yl]-N,N-diisopropylbenzamide, (R)-(+)-1-(4-bromobenzyl)-4-[(3-cyclopentyloxy)-4-methoxyphenyl]-2-pyrrolidone, 3-(cyclopentyloxy-4-methoxyphenyl)-1-(4-N'-[N-2-cyano-S-methyl-isothioureido]benzyl)-2-pyrrolidone, cis[4-cyano-4-(3-cyclopentyloxy-4-methoxyphenyl)cyclohexane-1-carbonic acid], 2-carbomethoxy-4-cyano-4-(3-cyclopropylmethoxy-4-difluoromethoxyphenyl)cyclohexan-1-one, cis[4-cyano-4-(3-cyclopropylmethoxy-4-difluoromethoxyphenyl)cyclohexan-1-ol], (R)- (+) -ethyl [4-(3-cyclopentyloxy-4-methoxyphenyl)pyrrolidin-2-yliden]acetate, (S)-(-)-ethyl[4-(3-cyclopentyloxy-4-methoxyphenyl)pyrrolidin-2-yliden]acetate, CDP840, Bay-198004, D-4418, PD-168787, T-440, T-2585, arofyllin, atizoram, V-11294A, Cl-1018, CDC-801, CDC-3052, D-22888, YM-58997, Z-15370, 9-cyclopentyl-5,6-dihydro-7-ethyl-3- (2-thienyl) -9*H*-pyrazolo [3, 4-c]-1, 2, 4-triazolo[4,3-a]pyridine and 9-cyclopentyl-5,6-dihydro-7-ethyl-3-(*tert*-butyl)-9*H*-pyrazolo[3,4-c]-1,2,4-triazolo[4,3-a]pyridine. These compounds may be used, as available, in the form of their racemates, enantiomers or diastereoisomers, or in the form of pharamacologically acceptable acid addition salts thereof, or in the form of their solvates and/or hydrates.

Steroids may preferably be selected from the group consisting of prednisolone, prednisone, butixocortpropionate, RPR-106541, flunisolid, beclomethasone, triamcinolone, budesonid, fluticasone, mometasone, ciclesonid, rofleponid, ST-126, dexamethasone, 6α,9α-difluoro-17α-[(2-furanylcarbonyl)oxy]-11β-hydroxy-16α-methyl-3-oxo-androsta-1,4-dien-17β-carbothionic acid (S)-fluoromethylester, and 6α,9α-difluoro-11β-hydroxy-16α-methyl-3-oxo-17α-propionyloxy-androsta-1,4-diene-17β-carbothionic acid (S)-(2-oxo-tetrahydro-furan-3S-yl)ester. These compounds may be used, as available, in the form of their racemates, enantiomers or diastereoisomers, or in the form of pharamacologically acceptable acid addition salts thereof, or in the form of their solvates and/or hydrates.

p38 MAP kinase inhibitors may preferably be selected from the group consisting of the p38 Kinase inhibitors that are disclosed for instance in US Patents 5,716,972, US 5,686,455, US 5,656,644, US 5,593,992, US 5,593,991, US 5,663,334, US 5,670,527, US 5,559,137, 5,658,903, US 5,739,143, US 5,756,499, US 6,277,989, US 6,340,685, and US 5,716,955 and PCT applications WO 92/12154, WO 94/19350, WO 95/09853, WO 95/09851, WO 95/09847, WO 95/09852, WO 97/25048, WO 97/25047, WO 97/33883, WO 97/35856, WO 97/35855, WO 97/36587, WO 97/47618, WO 97/16442, WO 97/16441, WO 97/12876, WO 98/25619, WO 98/06715, WO 98/07425, WO 98/28292, WO 98/56377, WO 98/07966, WO 98/56377, WO 98/22109, WO 98/24782, WO 98/24780, WO 98/22457, WO 98/52558, WO 98/52559, WO 98/52941, WO 98/52937, WO 98/52940, WO 98/56788, WO 98/27098, WO 98/47892, WO 98/47899, WO 98/50356, WO 98/32733, WO 99/58523, WO 99/01452, WO 99/01131, WO 99/01130, WO 99/01136, WO 99/17776, WO 99/32121, WO 99/58502, WO 99/58523, WO 99/57101, WO 99/61426, WO 99/59960, WO 99/59959, WO 99/00357, WO 99/03837, WO 99/01441, WO 99/01449, WO 99/03484, WO 99/15164, WO 99/32110, WO 99/32111, WO 99/32463, WO 99/64400, WO 99/43680, WO 99/17204, WO 99/25717, WO 99/50238, WO 99/61437, WO 99/61440, WO 00/26209, WO 00/18738, WO 00/17175, WO 00/20402, WO 00/01688, WO 00/07980, WO 00/07991, WO 00/06563, WO 00/12074, WO 00/12497, WO 00/31072, WO 00/31063, WO 00/23072, WO 00/31065, WO 00/35911, WO 00/39116, WO 00/43384, WO 00/41698, WO 00/69848, WO 00/26209, WO 00/63204, WO 00/07985, WO 00/59904, WO 00/71535, WO 00/10563, WO 00/25791, WO 00/55152, WO 00/55139, WO 00/17204, WO 00/36096, WO 00/55120, WO 00/55153, WO 00/56738, WO 01/21591, WO 01/29041, WO 01/29042, WO 01/62731, WO 01/05744, WO 01/05745, WO 01/05746, WO 01/05749, WO 01/05751, WO 01/27315, WO 01/42189, WO 01/00208, WO 01/42241, WO 01/34605, WO 01/47897, WO 01/64676, WO 01/37837, WO 01/38312, WO 01/38313, WO 01/36403, WO 01/38314, WO 01/47921, WO 01/27089, DE 19842833, and JP 2000 86657 whose disclosures are all incorporated herein by reference in their entirety. Of particular interest for the combinations according to the invention are those p38 inhibitors disclosed in US 6,277,989, US 6,340,685, WO 00/12074, WO 00/12497, WO 00/59904, WO 00/71535, WO 01/64676, WO 99/61426, WO 00/10563, WO 00/25791, WO 01/37837, WO 01/38312, WO 01/38313, WO 01/38314, WO 01/47921, WO 99/61437, WO 99/6144.0, WO 00/17175, WO 00/17204, WO 00/36096, WO 98/27098, WO 99/00357, WO 99/58502, WO 99/64400, WO 99/01131, WO 00/43384, WO 00/55152, WO 00/55139, and WO 01/36403. In a preferred embodiment the p38 kinase inhibitor is selected from the compounds of following formula (I) as disclosed in WO 99/01131 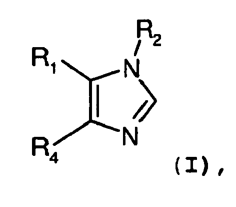 wherein
R₁ is 4-pyridyl, pyrimidinyl, 4-pyridazinyl, 1,2,4-triazin-5-yl, quinolyl, isoquinolinyl, or quinazolin-4-yl ring, which ring is substituted with Y-Rₐ and optionally with an additional independent substituent selected from C₁₋₄ alkyl, halogen, hydroxyl, C₁₋₄ alkoxy, C₁₋₄ akylthio, C₁₋₄ aklylsulfinyl, CH₂OR₁₂, amino, mono and di- C₁₋₆ alkyl substituted amino, an N-heterocyclyl ring which ring has from 5 to 7 members and optionally contains an additional heteroatom selected from oxygen, sulfur or NR₁₅, N (R₁₀) C (O) R_{b} or NHRₐ;
Y is oxygen or sulfur;
R₄ is phenyl, naphth-1-yl or naphthyl, or a heteroaryl, which is optionally substituted by one or two substituents, each of which is independently selected, and which, for a 4-phenyl, 4naphth-1-yl, 5-naphth-2-yl or 6-naphth-2-yl substituent, is halogen, cyano, nitro, C(Z)NR₇R₁₇, C((Z)) OR₁₆, (CR₁₀R₂₀)ᵥCOR₁₂, SR₅, SOR₅, OR₁₂, halo-substituted-C₁₋₄ alkyl, C₁₋₄ alkyl, ZC(Z)R₁₂, NR₁₀C(Z) R₁₆, or (CR₁₀R₂₀)ᵥNR₁₀R₂₀ and which, for other positions of substitution, is halogen, cyano, C(Z)NR₁₃R₁₄, C(Z)OR₃, (CR₁₀R₂₀)_{m"}COR₃, S(O)ₘR₃, OR₃, halo-substituted-C₁₋₄ alkyl, C₁₋₄ alkyl, (CR₁₀R₂₀)_{m"}R₁₀C(Z)R₃, NR₁₀S(O)_{m'}R₈, NR₁₀S(O)ₘNR₇R₁₇, ZC(Z)R₃ or (CR₁₀R₂₀)_{m"}NR₁₃R₁₄;
Z is oxygen or sulfur;
n is an integer having a value of 1 to 10;
m is 0, or integer 1 or 2;
m' is an integer having a value of 1 or 2;
m" is 0, or an integer having a value of 1 to 5;
v is 0, or an integer having a value of 1 to 2;
R₂ is -C(H) (A) (R₂₂);
A is optionally substituted aryl, heterocyclyl, or heteroaryl ring, or A is substituted C₁₋₁₀ alkyl;
R₂₂ is an optionally substituted C₁₋₁₀ alkyl;
Rₐ is aryl, arylC₁₋₆ alkyl, heterocyclic, heterocyclylC₁₋₆ alkyl, heteroaryl, heteroarylC₁₋₆alkyl, wherein each of these moieties may be optionally substituted;
R_{b} is hydrogen, C₁₋₆ alkyl, C₃₋₇ cycloalkyl, aryl, aryl C₁₋₄ alkyl, heteroaryl, heteroarylC₁₋₄ alkyl, heterocyclyl, or heterocyclylC₁₋₄ alkyl, wherein each of these moieties may be optionally substituted;
R₃ is heterocyclyl, heterocyclyl C₁₋₁₀ alkyl or R₈;
R₅ is hydrogen, C₁₋₄ alkyl, C₂₋₄ alkenyl, C₂₋₄ alkynyl or NR₇R₁₇, excluding the moieties SR₅ being SNR₇R₁₇ and Sorts bering SOH;
R₆ is hydrogen, a pharmaceutically acceptable cation, C₁₋₁₀ alkyl, C₃₋₇ cycloalkyl, aryl, aryl C₁₋₄ alkyl, heteroaryl, heteroaryl C₁₋₄ alkyl, heterocyclyl, aryl, or C₁₋₁₀alkanoyl; R₇ and R₁₇ is each independently selected from hydrogen or C₁₋₄ alkyl or R₇ and R₁₇ together with the nitrogen to which they are attached form a heterocyclic ring of 5 to 7 members which ring.optionally contains an additional heteroatom selected from oxygen, sulfur or NR₁₅;
R₈ is C₁₋₁₀ alkyl, halo-substituted C₁₋₁₀ alkyl, C₂₋₁₀ alkenyl, C₂₋₁₀ alkynyl, C₃₋₇ cycloalkyl, C₅₋₇ cycloalkenyl, aryl, aryl C₁₋₁₀ alkyl, heteroaryl, heteroaryl C₁₋₁₀ alkyl, (CR₁₀R₂₀)ₙ OR₁₁, (CR₁₀R₂₀)ₙS (O)ₘR₁₈, (CR₁₀R₂₀) ₙNHS (O) ₂R₁₈, (CR₁₀R₂₀)ₙNR₁₃R₁₄; wherein the aryl, arylalkyl, heteroaryl, heteroaryl alkyl may be optionally substituted;
R₉ is hydrogen, C (Z) R₁₁ or optionally substituted C₁₋₁₀ alkyl, S(O)₂R₁₈, optionally substituted aryl or optionally substituted aryl C₁₋₄ alkyl;
R₁₀ and R₂₀ is each independently selected from hydrogen or C₁₋₄ alkyl;
R₁₁ is hydrogen, C₁₋₁₀ alkyl, C₃₋₇ cycloalkyl, heterocyclyl, heterocyclyl C₁₋₁₀ alkyl, aryl, arylC₁₋₁₀ alkyl, heteroaryl or heteroaryl C₁₋₁₀ alkyl, wherein these moieties may be optionally substituted;
R₁₂ is hydrogen or R₁₆;
R₁₃ an R₁₄ is each independently selected from hydrogen or optionally substituted
C₁₋₄ alkyl, optionally substituted aryl or optionally substituted arylC₁₋₄ alkyl, or together with the nitrogen which they are attached form a heterocyclic ring of 5 to 7 members which ring optionally contains an additional heteroatom selected from oxygen, sulfur or NR₉;
R₁₅ is R₁₀ or C(Z)-C₁₋₄ alkyl;
R₁₆ is C₁₋₄ alkyl, halo-substituted-C₁₋₄ alkyl, or C₃₋₇ cycloalkyl;
R₁₈ is C₁₋₁₀ alkyl, C₃₋₇ cycloalkyl, heterocyclyl, aryl, aryl₁₋₁₀ alkyl, heterocyclyl, heterocyclyl- C₁₋₁₀alkyl, heteroaryl or heteroaryl₁₋₁₀ alkyl;
or a pharmaceutically acceptable salt thereof.

NK₁ antagonists may preferably be selected from the group consisting of N-[2-(3,5-bis-trifluoromethyl-phenyl)-ethyl]-2-{4-cyclopropylmethyl-piperazin-1-yl}-N-methyl-2-phenyl-acetamide (BIIF 1149), CP-122721, FK-888, NKP 608C, NKP 608A, CGP 60829, SR 48968 (Saredutant), SR 140333 (Nolpitantium besilate/chloride), LY 303 870 (Lanepitant), MEN-11420 (Nepadutant), SB 223412, MDL-105172A, MDL-103896, MEN-11149, MEN-11467, DNK 333A, SR-144190, YM-49244, YM-44778, ZM-274773, MEN-10930, S-19752, Neuronorm, YM-35375, DA-5018, Aprepitant (MK-869), L-754030, CJ-11974, L-758298, DNK-33A, 6b-I, CJ-11974, TAK-637, GR 205171 and the arylglycine amide derivates of general formula **(VIII)** 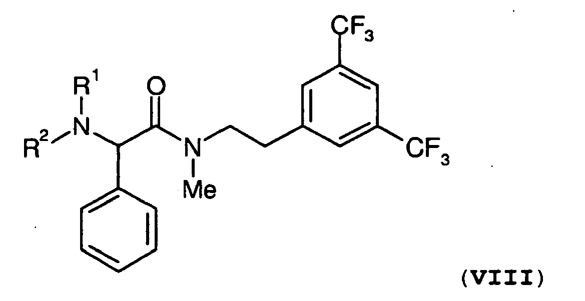 wherein
R¹ and R² together with the N-atom they are bound to form a ring of formula 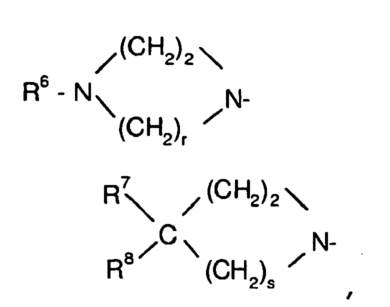 or
wherein r and s independently denote the number 2 or 3;
R⁶ denotes H, -C₁-C₅-alkyl, C₃-C₅-alkenyl, propinyl, hydroxy (C₂-C₄) alkyl, methoxy (C₂-C₄) alkyl, di (C₁-C₃) alkylamino (C₂-C₄) alkyl, amino (C₂-C₄) alkyl, amino, di (C₁-C₃)alkylamino, monofluoro- up to perfluoro(C₁-C₂)alkyl, N-methylpiperidinyl, pyridyl, pyrimidinyl, pyrazinyl or pyridazinyl,
R⁷ denotes any of the groups defined under (a) to (d):
(a) hydroxy
(b) 4-piperidinopiperidyl,
(c) 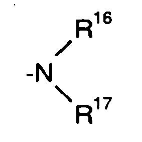 wherein R¹⁶ and R¹⁷ independently denote
H, (C₁-C₄) alkyl, (C₃-C₆)cycloalkyl, hydroxy (C₂-C₄) alkyl, dihydroxy (C₂-C₄) alkyl, (C₁-C₃) alkoxy (C₂-C₄) alkyl, phenyl(C₁-C₄)alkyl or di(C₁-C₃)alkylamino(C₂-C₄)alkyl, and
R⁸ denotes H,
optionally in the form of enantiomers, mixtures of enantiomers or the racemates.

The compounds of formula **(VIII)** mentioned hereinbefore are described in WO 96/32386, WO 97/32865 and WO 02/32865. The disclosure of these international patent applications is incorporated herein by reference in its entirety.

LTD4 antagonists may preferably be selected from the group consisting of montelukast, 1-( ( (R)- (3- (2-(6,7-difluoro-2-quinolinyl)ethenyl)phenyl)-3-(2-(2- hydroxy-2-propyl)phenyl)thio)methylcyclopropane-acetate, 1-(((1(R)-3 (3- (2- (2, 3-dichlorothieno [3, 2-b]pyridin-5-yl)- (E) - ethenyl)phenyl)-3-(2-(1-hydroxy-1-methylethyl)phenyl)propyl)thio)methyl)cyclopropane-acetate, pranlukast, zafirlukast, [2-[[2-(4-tert-butyl-2-thiazolyl)-5-benzofuranyl]oxymethyl]phenyl]acetate, MCC-847 (ZD-3523), MN-001, MEN-91507 (LM-1507), VUF-5078, VUF-K-8707 and L-733321. These compounds may be used, as available, in the form of their racemates, enantiomers or diastereoisomers, or in the form of pharamacologically acceptable acid addition salts thereof, or in the form of their solvates and/or hydrates.

EGFR inhibitors may preferably be selected from the group consisting of 4-[(3-chlor-4-fluorphenyl)amino]-6-{[4-(morpholin-4-yl)-1-oxo-2-buten-1-yl]amino}-7-cyclopropylmethoxy-chinazoline, 4-[(3-chlor-4-fluorphenyl)amino]-6-{[4-(N,N-diethylamino)-1-oxo-2-buten-1-yl]amino}-7-cyclopropylmethoxy-chinazoline, 4-[(3-chlor-4-fluorphenyl)amino]-6-{[4-(N,N-dimethylamino)-1-oxo-2-buten-1-yl]amino}-7-cyclopropylmethoxy-chinazoline, 4-[(*R*)-(1-phenyl-ethyl)amino]-6-{[4-(morpholin-4-yl)-1-oxo-2-buten-1-yl]amino}-7-cyclopentyloxy-chinazoline, 4-[(3-chlor-4-fluor-phenyl)amino]-6-{[4-((*R*)-6-methyl-2-oxo-morpholin-4-yl)-1-oxo-2-buten-1-yl]amino}-7-cyclopropylmethoxy-chinazoline, 4-[(3-chlor-4-fluorphenyl) amino]-6-{[4-((*R*)-6-methyl-2-oxo-morpholin-4-yl) -1-oxo-2-buten-1-yl]amino}-7-[(*S*)-(tetrahydrofuran-3-yl)oxy]-chinazoline, 4-[(3-chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazoline, 4-[(3-color-4-fluor-phenyl)amino]-6-[2-((*S*)-6-methyl-2-oxo-morpholin-4-yl)-ethoxy]-7-methoxy-chinazoline, 4-[(3-chlor-4-fluorphenyl)amino]-6-({4-[N-(2-methoxy-ethyl)-N-methyl-amino]-1-oxo-2-buten-1-yl}amino)-7-cyclopropylmethoxy-chinazoline, 4-[(3-chlor-4-fluorphenyl)amino]-6-{[4-(N,N-dimethylamino)-1-oxo-2-buten-1-yl]amino}-7-cyclopentyloxy-chinazoline, 4-[(*R*)-(1-phenyl-ethyl)amino]-6-{[4-(N,N-bis-(2-methoxy-ethyl)-amino)-1-oxo-2-buten-1-yl]amino}-7-cyclopropylmethoxy-chinazoline, 4-[(*R*)-(1-phenylethyl)amino]-6-({4-[N-(2-methoxy-ethyl)-N-ethyl-amino]-1-oxo-2-buten-1-yl}amino)-7-cyclopropylmethoxy-chinazoline, 4-[(*R*)-(1-phenyl-ethyl)amino]-6-({4-[N-(2-methoxy-ethyl)-N-methyl-amino]-1-oxo-2-buten-1-yl}amino)-7-cyclopropylmethoxy-chinazoline, 4-[(*R*)-(1-phenylethyl)amino]-6-({4-[N-(tetrahydropyran-4-yl)-N-methyl-amino]-1-oxo-2-buten-1-yl}amino)-7-cyclopropylmethoxy-chinazoline, 4-[(3-chlor-4-fluorphenyl)amino]-6-{[4-(N,N-dimethylamino)-1-oxo-2-buten-1-yl]amino}-7-((*R*)-tetrahydrofuran-3-yloxy)-chinazoline, 4-[(3-chlor-4-fluorphenyl) amino]-6-{[4-(N,N-dimethylamino)-1-oxo-2-buten-1-yl]amino}-7-((*S*)-tetrahydrofuran-3-yloxy)-chinazoline, 4-[(3-chlor-4-fluorphenyl)amino]-6-({4-[N-(2-methoxy-ethyl)-N-methyl-amino]-1-oxo-2-buten-1-yl}amino)-7-cyclopentyloxy-chinazoline, 4-[(3-chlor-4-fluorphenyl)amino]-6-{[4-(N-cyclopropyl-N-methyl-amino)-1-oxo-2-buten-1-yl]amino}-7-cyclopentyloxy-chinazoline, 4-[(3-chlor-4-fluorphenyl)amino]-6-{[4-(N,N-dimethylamino)-1-oxo-2-buten-1-yl] amino}-7-[(*R*)-(tetrahydrofuran-2-yl) methoxy]-chinazoline, 4-[(3-chlor-4-fluorphenyl)amino]-6-{[4-(N,N-dimethylamino)-1-oxo-2-buten-1-yl]amino}-7-[(*S*)-(tetrahydrofuran-2-yl)methoxy]-chinazoline, 4-[(3-ethinylphenyl)amino]-6,7-bis-(2-methoxy-ethoxy)-chinazoline, 4-[(3-chlor-4-fluorphenyl)amino]-7-[3-(morpholin-4-yl)-propyloxy]-6-[(vinylcarbonyl)amino]-chinazoline, 4-[(*R*)-(1-phenyl-ethyl)amino]-6-(4-hydroxy-phenyl)-7H-pyrrolo[2,3-d]pyrimidine, 3-cyano-4-[(3-chlor-4-fluorphenyl)amino]-6-{[4-(N,N-dimethylamino)-1-oxo-2-buten-1-yl]amino}-7-ethoxy-chinoline, 4-{[3-chlor-4-(3-fluor-benzyloxy)-phenyl]amino}-6-(5-{[(2-methansulfonyl-ethyl)amino]methyl}-furan-2-yl) chinazoline, 4-[(R)-(1-phenyl-ethyl)amino]-6-{[4-((R)-6-methyl-2-oxo-morpholin-4-yl)-1-oxo-2-buten-1-yl]amino}-7-methoxy-chinazoline, 4-[(3-chlor-4-fluorphenyl)amino]-6-{[4-(morpholin-4-yl)-1-oxo-2-buten-1-yl]amino}-7-[(tetrahydrofuran-2-yl)methoxy]-chinazoline, 4-[(3-chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazoline, 4-[(3-ethinyl-phenyl)amino]-6-{[4-(5,5-dimethyl-2-oxo-morpholin-4-yl)-1-oxo-2-buten-1-yl]amino}-chinazoline, 4-[(3-chlor-4-fluor-phenyl)amino]-6-[2-(2,2-dimethyl-6-oxo-morpholin-4-yl)-ethoxy]-7-methoxy-chinazoline, 4-[(3-chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazoline, 4-[(3-chlor-4-fluor-phenyl) amino]-7- [2- (2,2-dimethyl-6-oxo-morpholin-4-yl)-ethoxy]-6-[(S)-(tetrahydrofuran-2-yl)methoxy]-chinazoline, 4-[(3-chlor-4-fluor-phenyl)amino]-6-{2-[4-(2-oxo-morpholin-4-yl)-piperidin-1-yl]-ethoxy}-7-methoxy-chinazoline, 4-[(3-chlor-4-fluor-phenyl)amino]-6-[1-(tert.-butyloxycarbonyl)-piperidin-4-yloxy]-7-methoxy-chinazoline, 4-[(3-chlor-4-fluor-phenyl)amino]-6-(trans-4-amino-cyclohexan-1-yloxy)-7-methoxy-chinazoline, 4-[(3-chlor-4-fluor-phenyl)amino]-6-(trans-4-methansulfonylamino-cyclohexan-1-yloxy)-7-methoxy-chinazoline, 4-[(3-chlor-4-fluor-phenyl)amino]-6-(tetrahydropyran-3-yloxy)-7-methoxy-chinazoline, 4-[(3-chlor-4-fluor-phenyl)amino]-6-(1-methyl-piperidin-4-yloxy)-7-methoxy-chinazoline, 4-[(3-chlor-4-fluor-phenyl)amino]-6-{1-[(morpholin-4-yl)carbonyl]-piperidin-4-yloxy}-7-methoxy-chinazoline, 4-[(3-chlor-4-fluor-phenyl)amino]-6-{1-[(methoxymethyl)carbonyl]-piperidin-4-yloxy}-7-methoxy-chinazoline, 4-[(3-chlor-4-fluor-phenyl)amino]-6-(piperidin-3-yloxy)-7-methoxy-chinazoline, 4-[(3-chlor-4-fluor-phenyl)amino]-6-[1-(2-acetylamino-ethyl)-piperidin-4-yloxy]-7-methoxy-chinazoline, 4-[(3-chlor-4-fluor-phenyl)amino]-6-(tetrahydropyran-4-yloxy)-7-ethoxy-chinazoline, 4-[(3-chlor-4-fluor-phenyl)amino]-6-((S)-tetrahydrofuran-3-yloxy)-7-hydroxy-chinazoline, 4-[(3-chlor-4-fluor-phenyl)amino]-6-(tetrahydropyran-4-yloxy)-7-(2-methoxy-ethoxy)-chinazoline, 4-[(3-chlor-4-fluor-phenyl)amino]-6-{trans-4-[(dimethylamino)sulfonylamino]-cyclohexan-1-yloxy}-7-methoxy-chinazoline, 4-[(3-chlor-4-fluor-phenyl)amino]-6-{trans-4-[(morpholin-4-yl)carbonylamino]-cyclohexan-1-yloxy}-7-methoxy-chinazoline, 4-[(3-chlor-4-fluor-phenyl)amino]-6-{trans-4-[(morpholin-4-yl)sulfonylamino]-cyclohexan-1-yloxy}-7-methoxy-chinazoline, 4-[(3-chlor-4-fluor-phenyl)amino]-6-(tetrahydropyran-4-yloxy)-7-(2-acetylamino-ethoxy)-chinazoline, 4-[(3-chlor-4-fluor-phenyl)amino]-6-(tetrahydropyran-4-yloxy)-7-(2-methansulfonylamino-ethoxy)-chinazoline, 4-[(3-chlor-4-fluor-phenyl)amino]-6-{1-[(piperidin-1-yl)carbonyl]-piperidin-4-yloxy}-7-methoxy-chinazoline, 4-[(3-chlor-4-fluor-phenyl)amino]-6-(1-aminocarbonylmethyl-piperidin-4-yloxy)-7-methoxy-chinazoline, 4-[(3-chlor-4-fluor-phenyl)amino]-6-(cis-4-{N-[(tetrahydropyran-4-yl)carbonyl]-N-methyl-amino}-cyclohexan-1-yloxy)-7-methoxy-chinazoline, 4-[(3-chlor-4-fluor-phenyl)amino]-6-(cis-4-{N-[(morpholin-4-yl)carbonyl]-N-methyl-amino}-cyclohexan-1-yloxy)-7-methoxy-chinazoline, 4-[(3-chlor-4-fluor-phenyl)amino]-6-(cis-4-{N-[(morpholin-4-yl)sulfonyl]-N-methyl-amino}-cyclohexan-1-yloxy)-7-methoxy- chinazoline, 4-[(3-chlor-4-fluor-phenyl)amino]-6-(trans-9-ethansulfonylamino-cyclohexan-1-yloxy)-7-methoxy-chinazoline, 4-[(3-chlor-4-fluor-phenyl)amino]-6-(1-methansulfonyl-piperidin-4-yloxy)-7-ethoxy-chinazoline, 4-[(3-chlor-4-fluor-phenyl)amino]-6-(1-methansulfonyl-piperidin-4-yloxy)-7-(2-methoxy-ethoxy)-chinazoline, 4-((3-chlor-4-fluor-phenyl)amino]-6-[1-(2-methoxy-acetyl)-piperidin-4-yloxy]-7-(2-methoxy-ethoxy)-chinazoline, 4-[(3-chlor-4-fluor-phenyl)amino]-6-(cis-4-acetylamino-cyclohexan-1-yloxy)-7-methoxy-chinazoline, 4-[(3-Ethinylphenyl)amino]-6-.[1-(tert.-butyloxycarbonyl)-piperidin-4-yloxy]-7-methoxy-chinazoline, 4-[(3-Ethinyl-phenyl)amino]-6-(tetrahydropyran-4-yloxy]-7-methoxy-chinazoline, 4-[(3-chlor-4-fluor-phenyl)amino]-6-(cis-4-{N-[(piperidin-1-yl)carbonyl]-N-methyl-amino}-cyclohexan-1-yloxy)-7-methoxy-chinazoline, 4-[(3-chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazoline, 4-[(3-chlor-4-fluor-phenyl)amino]-6-{cis-4-[(morpholin-4-yl)carbonylamino]-cyclohexan-1-yloxy}-7-methoxy-chinazoline, 4-[(3-chlor-4-fluor-phenyl)amino]-6-{1-[2-(2-oxopyrrolidin-1-yl)ethyl]-piperidin-4-yloxy}-7-methoxy-chinazoline, 4-[(3-chlor-4-fluor-phenyl)amino]-6-{1-[(morpholin-4-yl)carbonyl]-piperidin-4-yloxy}-7-(2-methoxy-ethoxy)-chinazoline, 4-[(3-Ethinyl-phenyl)amino]-6-(1-acetyl-piperidin-4-yloxy)-7-methoxy-chinazoline, 4-[(3-Ethinyl-phenyl)amino]-6-(1-methyl-piperidin-4-yloxy)-7-methoxy-chinazoline, 4-[(3-Ethinyl-phenyl)amino]-6-(1-methansulfonyl-piperidin-4-yloxy)-7-methoxy-chinazoline, 4-[(3-chlor-4-fluor-phenyl)amino]-6-(1-methyl-piperidin-4-yloxy)-7(2-methoxy-ethoxy)-chinazoline, 4-[(3-chlor-4-fluor-phenyl)amino]-6-(1-isopropyloxycarbonyl-piperidin-4-yloxy)-7-methoxy-chinazoline, 4-[(3-chlor-4-fluorphenyl)amino]-6-(cis-4-methylamino-cyclohexan-1-yloxy)-7-methoxy-chinazoline, 4-[(3-chlor-4-fluor-phenyl)amino]-6-{cis-4-[N-(2-methoxy-acetyl)-N-methyl-amino]-cyclohexan-1-yloxy}-7-methoxy-chinazoline, 4-[(3-Ethinyl-phenyl)amino]-6-(piperidin-4-yloxy)-7-methoxy-chinazoline, 4-[(3-Ethinyl-phenyl)amino]-6-[1-(2-methoxy-acetyl)-piperidin-4-yloxy]-7-methoxy-chinazoline, 4-[(3-Ethinyl-phenyl)amino]-6-{1-[(morpholin-4-yl)carbonyl]-piperidin-4-yloxy}-7-methoxy-chinazoline, 4-[(3-chlor-4-fluor-phenyl)amino]-6-{1-[(cis-2,6-dimethyl-morpholin-4-yl)carbonyl]-piperidin-4-yloxy}-7-methoxy-chinazoline, 4-[(3-chlor-4-fluor-phenyl)amino]-6-{1-[(2-methyl-morpholin-4-yl)carbonyl]-piperidin-4-yloxy}-7-methoxy-chinazoline, 4-[(3-chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazoline, 4-[(3-chlor-4-fluor-phenyl)amino]-6-{1-[(N-methyl-N-2-methoxyethyl-amino)carbonyl]-piperidin-4-yloxy}-7-methoxy-chinazoline, 4-[(3-chlor-4-fluor-phenyl)amino]-6-(1-ethyl-piperidin-4-yloxy)-7-methoxy-chinazoline, 4-[(3-chlor-4-fluorphenyl)amino]-6-{1-[(2-methoxyethyl)carbonyl]-piperidin-4-yloxy}-7-methoxy-chinazoline, 4-[(3-chlor-4-fluorphenyl)amino]-6-{1-[(3-methoxypropyl-amino)-carbonyl]-piperidin-4-yloxy}-7-methoxy-chinazoline, 4-[(3-chlor-4-fluor-phenyl)amino]-6-[cis-4-(N-methansulfonyl-N-methyl-amino)-cyclohexan-1-yloxy]-7-methoxy-chinazoline, 4-[(3-chlor-4-fluor-phenyl)amino]-6-[cis-4-(N-acetyl-N-methyl-amino)-cyclohexan-l-yloxy]-7-methoxy-chinazoline, 4-[(3-chlor-4-fluor-phenyl)amino]-6-(trans-4-methylaminocyclohexan-1-yloxy)-7-methoxy-chinazoline, 4-[(3-chlor-4-fluor-phenyl)amino]-6-[trans-4-(N-methansulfonyl-N-methyl-amino)-cyclohexan-1-yloxy]-7-methoxy-chinazoline 4-[(3-chlor-4-fluor-phenyl)amino]-6-(trans-4-dimethylaminocyclohexan-1-yloxy)-7-methoxy-chinazoline , 4-[(3-chlor-4-fluor-phenyl)amino]-6-(trans-4-{N-[(morpholin-4-yl)carbonyl]-N-methyl-amino}-cyclohexan-1-yloxy)-7-methoxy-chinazoline, 4-[(3-chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazoline, 4-[(3-chlor-4-fluor-phenyl)amino]-6-(1-methansulfonyl-piperidin-4-yloxy)-7-methoxy-chinazoline, 4-[(3-chlor-4-fluor-phenyl)amino]-6-(1-cyano-piperidin-4-yloxy)-7-methoxy-chinazoline, Cetuximab, Trastuzumab, ABX-EGF and Mab ICR-62. These compounds may be used, as available, in the form of their racemates, enantiomers or diastereoisomers, or in the form of pharamacologically acceptable acid addition salts thereof, or in the form of their solvates and/or hydrates. These compounds are disclosed in the prior art, e.g. in WO 96/30347, WO 97/02266, WO 99/35146, WO 00/31048, WO 00/78735, WO 01/34574, WO 01/61816, WO 01/77104, WO02/18351, WO 02/18372, WO 02/18373, WO 02/18376, WO 02/50043, WO 03/082290, Cancer Research 2004, 64:11 (3958-3965), Am J Health-Syst Pharm 2000, 57(15), 2063-2076, Clinical Therapeutics 1999, 21(2), 309-318, WO 98/50433, and WO 95/20045.

Endothelin-antagonists may preferably be selected from the group consisting of tezosentan, bosentan, enrasentan, sixtasentan, T-0201, BMS-193884, K-8794, PD-156123, PD-156707, PD-160874, PD-180988, S-0139 and ZD-1611. Any reference to endothelin-antagonists within the scope of the present invention includes a reference to the salts, preferably pharmacologically acceptable acid addition salts, or derivatives which may be formed from the endothelin-antagonists.

These combinations may be administered either simultaneously or sequentially.

For pharmaceutical use the compounds according to the invention are preferably used for warm-blooded vertebrates, particularly humans, in doses of 0.0001-100 mg/kg of body weight.

These compounds may be administered either on their own or in conjunction with other active substances by intravenous, subcutaneous, intramuscular, intraperitoneal or intranasal route, by inhalation, or transdermally, or orally, whilst aerosol formulations are particularly suitable for inhalation.

For administration they are formulated with one or more conventional inert solid, semisolid or liquid carriers e.g. with starch, different types of cellulose, lactose, mannitol, sorbitol, glucose, calcium phosphate, hard fat, fatty alcohols, glycerol, medium chained triglycerides and related esters, polyethylene glycol, refined specialty oils, water, water/ethanol, water/glycerol, water/sorbitol, water/polyethylene glycol, propylene glycol,and/or functional excipients, e.g. with polyvinylpyrrolidone, hydroxypropylmethylcellulose, sodium carboxymethylcellulose, sodium starch glycolate, silicon dioxide, polysorbates, poloxamers, gelucires, magnesium stearate, citric acid, tartaric acid, or suitable mixtures thereof in conventional galenic preparations such as plain or coated tablets, capsules, powders, injectable solutions, ampoules; suspensions, solutions, sprays or suppositories.

The following examples of formulations illustrate the present invention without representing a limitation of its scope.

### Example F1: Coated tablet containing 75 mg of active substance Composition

1 tablet core contains:

| | |
|---|---|
| active substance | 75.0 mg |
| calcium phosphate | 131.0 mg |
| polyvinylpyrrolidone | 10.0 mg |
| carboxymethylcellulose sodium | 10.0 mg |
| silicon dioxide | 2.5 mg |
| magnesium stearate | 1.5 mg |
| | 230.0 mg |

### Preparation (direct compression)

The active substance is mixed with all components, sieved and compressed in a tablet-making machine to form tablets of the desired shape.

| | |
|---|---|
| Weight of core: | 230 mg |
| Appearance of core: | 9 mm, biconvex |

The tablet cores thus produced are coated with a film consisting essentially of hydroxypropylmethylcellulose.

| | |
|---|---|
| Weight of coated tablet: | 240 mg. |

### Example F2: Tablet containing 100 mg of active substance Composition

1 tablet contains:

| | |
|---|---|
| active substance | 100.0 mg |
| lactose | 80.0 mg |
| corn starch | 34.0 mg |
| hydroxypropylmethylcellulose | 4.0 mg |
| magnesium stearate | 2.0 mg |
| | 220.0 mg |

### Preparation (wet granulation)

The active substance, lactose and starch are mixed together and uniformly moistened with an aqueous solution of the hydroxypropylmethylcellulose. After the moist composition has been screened (2.0 mm mesh size) and dried in a rack-type drier at 50°C it is screened again (1.5 mm mesh size) and the lubricant is added. The finished mixture is compressed to form tablets.

| | |
|---|---|
| Weight of tablet: | 220 mg |
| Appearance of tablet: | 10 mm, flat faced with bevelled edges and breaking notch on one side. |

### Example F3: Tablet containing 150 mg of active substance Composition

1 tablet contains:

| | |
|---|---|
| active substance | 150.0 mg |
| lactose | 85.0 mg |
| microcrystalline cellulose | 40.0 mg |
| polyvinylpyrrolidone | 10.0 mg |
| silicon dioxide | 10.0 mg |
| magnesium stearate | 5.0 mg |
| | 300.0 mg |

### Preparation (dry granulation)

The active substance mixed with lactose, polyvinylpyrrolidone,and parts of the microcrystalline cellulose, magnesium stearate is compacted e.g. on a roller compactor. The ribbons are broken up in fine granules through a screen with a mesh size of 0.8 mm. After subsequent sieving through a screen with a mesh size of 0.5 mm and blending with the remaining components, tablets are pressed from the mixture.

| | |
|---|---|
| Weight of tablet: | 300 mg |
| Appearance of tablet: | 10 mm, flat |

### Example F4: Hard gelatine capsule containing 150 mg of active substance Composition

### 1 capsule contains:

| | |
|---|---|
| active substance | 150.0 mg |
| lactose | 85.0 mg |
| microcrystalline cellulose | 40.0 mg |
| polyvinylpyrrolidone | 10.0 mg |
| silicon dioxide | 10.0 mg |
| magnesium stearate | 5.0 mg |
| | 300.0 mg |

### Preparation

The active substance mixed with lactose, polyvinylpyrrolidone,and parts of the microcrystalline cellulose, magnesium stearate is compacted e.g. on a roller compactor. The ribbons are broken up in fine granules through a screen with a mesh size of 0.8 mm. After subsequent sieving through a screen with a mesh size of 0.5 mm and blending with the remaining components, the finished mixture is packed into size 1 hard gelatine capsules.
Capsule filling: approx. 300 mg
Capsule shell: size 1 hard gelatine capsule.

### Example F5: Suppository containing 150 mg of active substance

1 suppository contains:

| | |
|---|---|
| active substance | 150.0 mg |
| polyethyleneglycol 1500 | 800.0 mg |
| polyethyleneglycol 6000 | 850.0 mg |
| polyoxyl 40 hydrogenated castor oil | 200.0 mg |
| | 2,000.0 mg |

### Preparation

After the suppository mass has been melted the active substance is homogeneously distributed therein and the melt is poured into chilled moulds.

### Example F6: Suspension containing 50 mg of active substance

100 ml of suspension contains

| | |
|---|---|
| active substance | 1.00 g |
| carboxymethylcellulose sodium | 0.10 g |
| methyl p-hydroxybenzoate | 0.05 g |
| propyl p-hydroxybenzoate | 0.01 g |
| glucose | 10.00 g |
| glycerol | 5.00 g |
| 70% sorbitol solution | 20.00 g |
| flavouring | 0.30 g |
| dist. water ad | 100 ml |

### Preparation

The distilled water is heated to 70°C. The methyl and propyl p-hydroxybenzoates together with the glycerol and sodium salt of carboxymethylcellulose are dissolved therein with stirring. The solution is cooled to ambient temperature and the active substance is added and homogeneously dispersed therein with stirring. After the sugar, the sorbitol solution and the flavouring have been added and dissolved, the suspension is evacuated with stirring to eliminate air.

Thus, 5 ml of suspension contains 50 mg of active substance.

### Example F7: Ampoule containing 10 mg active substance Composition

| | |
|---|---|
| active substance | 10.0 mg |
| 0.01 N hydrochloric acid | q.s. |
| double-distilled water | ad 2.0 ml |

### Preparation

The active substance is dissolved in the necessary amount of 0.01 N HCl, made isotonic with sodium chloride, filtered sterile and transferred into a 2 ml ampoule.

### Example F8: Ampoule containing 50 mg of active substance Composition

| | |
|---|---|
| active substance | 50.0 mg |
| 0.01 N hydrochloric acid | q.s. |
| double-distilled water | ad 10.0 ml |

### Preparation

The active substance is dissolved in the necessary amount of 0.01 N HCl, made isotonic with sodium chloride, filtered sterile and transferred into a 10 ml ampoule.

### Example F9: Capsule for powder inhalation containing 5 mg of active substance

1 capsule contains

| | |
|---|---|
| active substance | 5.0 mg |
| lactose for inhalation | 15.0 mg |
| | 20.0 mg |

### Preparation

The active substance is mixed with lactose for inhalation. The mixture is packed into capsules in a capsule-making machine (weight of the empty capsule approx. 50 mg).
weight of capsule: 70.0 mg
size of capsule = size 3

### Example F10: Solution for inhalation for a hand-held nebuliser containing 2.5 mg active substance

1 spray contains

| | |
|---|---|
| active substance | 2.500 mg |
| benzalkonium chloride | 0.001 mg |
| 1N hydrochloric acid q.s. ethanol/water (50/50) | ad 15.000 mg |

### Preparation

The active substance and benzalkonium chloride are dissolved in ethanol/water (50/50). The pH of the solution is adjusted with 1N hydrochloric acid. The resulting solution is filtered and transferred into suitable containers for use in hand-held nebulisers (cartridges).

Contents of the container: 4.5 g

## Claims

1. Use of the indolinones of general formula 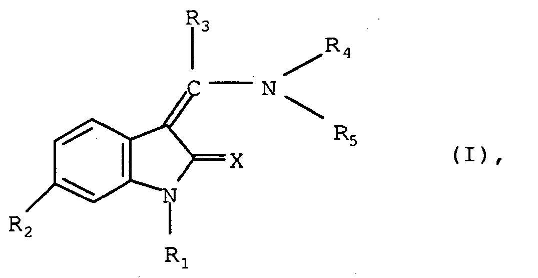 substituted in the 6 position, wherein
X denotes an oxygen or sulphur atom,
R₁ denotes a hydrogen atom or a prodrug group,
R₂ denotes a carboxy group, a straight-chain or branched C₁₋₆-alkoxycarbonyl group, a C₄₋₇-cycloalkoxycarbonyl or an aryloxycarbonyl group,
R₃ denotes a hydrogen atom, a C₁₋₆-alkyl, C₃₋₇-cycloalkyl, trifluoromethyl or heteroaryl group,
a phenyl or naphthyl group, or a phenyl or naphthyl group mono- or disubstituted by a fluorine, chlorine, bromine or iodine atom, by a trifluoromethyl, C₁₋₃-alkyl or C₁₋₃-alkoxy group, while in the event of disubstitution the substituents may be identical or different,
R₄ denotes a phenyl, pyrrolyl or furanyl group substituted by the group R₆, which may additionally be mono- or disubstituted by fluorine, chlorine, bromine or iodine atoms, by C₁₋₅-alkyl, trifluoromethyl, hydroxy, C₁₋₃-alkoxy, carboxy, C₁₋₃-alkoxycarbonyl, amino, acetylamino, C₁₋₃-alkyl-sulphonylamino, aminocarbonyl, C₁₋₃-alkyl-aminocarbonyl, di-(C₁₋₃-alkyl)-aminocarbonyl, aminosulphonyl, C₁₋₃-alkyl-aminosulphonyl, di-(C₁₋₃-alkyl)-aminosulphonyl, nitro or cyano groups, while the substituents may be identical or different and wherein
R₆ denotes an aminocarbonyl, C₁₋₄-alkylamino-carbonyl, N-(C₁₋₅-alkyl)-C₁₋₃-alkylaminocarbonyl, C₃₋₇-cycloalkylamino-carbonyl, N-(C₁₋₅-alkyl)-C₃₋₇-cycloalkylaminocarbonyl, (phenyl-C₁₋₃-alkyl)amino-carbonyl, N-(C₁₋₃-alkyl)-phenyl-C₁₋₃-alkylamino-carbonyl group,
a C₁₋₃-alkylaminocarbonyl or N-(C₁₋₃-alkyl)-C₁₋₃-alkylaminocarbonyl group wherein one or two alkyl moieties are substituted independently of one another by a nitro, cyano, carbamoyl, N-(C₁₋₃-alkyl)-carbamoyl, di-N-(C₁₋₃-alkyl)-carbamoyl, carboxy or C₁₋₃-alkoxycarbonyl group or are substituted in the 2- or 3-position by an amino, (C₁₋₃-alkyl)-amino, di-(C₁₋₃-alkyl)-amino, (C₁₋₄-alkoxycarbonyl)-amino, N-(C₁₋₄-alkoxycarbonyl) -N-(C₁₋₃-alkyl)-amino, piperazino, N-(C₁₋₃-alkyl)-piperazino, a 4- to 7-membered cycloalkyleneimino group, a hydroxy or methoxy group,
a 4- to 7-membered cycloalkyleneiminocarbonyl group wherein
the cycloalkylene moiety may be fused to a phenyl ring via two adjacent ring atoms or may form a bridge to a methylene or ethylene group via two non-adjacent ring atoms or
one or two hydrogen atoms may each be replaced by a C₂₋₃-alkyl group and/or
in each case the methylene group in the 4 position of a 6- or 7-membered cycloalkyleneiminocarbonyl group may be substituted by a carboxy, C₁₋₄-alkoxycarbonyl, aminocarbonyl, C₁₋₃-alkylaminocarbonyl, di-(C₁₋₃-alkyl) -aminocarbonyl, di-(C₁₋₃-alkyl)-amino-C₁₋₃-alkyl, di- (C₁₋₃-alkyl)-amino, phenyl-C₁₋₃-alkylamino or N-(C₁₋₃-alkyl)-phenyl-C₁₋₃-alkylamino group or a hydroxy or methoxy group or
may be replaced by an oxygen or sulphur atom, by a sulphinyl, sulphonyl for -NH group or by a nitrogen atom which is substituted by a C₁₋₃-alkyl, phenyl, C₁₋₃-alkyl-carbonyl, C₁₋₄-alkoxy-carbonyl, di-(C₁₋₃-alkyl)-amino-C₁₋₃-alkyl, hydroxy-C₂₋₃-alkyl or benzoyl group,
while all the single-bonded or fused phenyl groups contained in the groups mentioned under R₆ may be mono-or disubstituted by fluorine, chlorine, bromine or iodine atoms, by C₁₋₅-alkyl, trifluoromethyl, hydroxy C₁₋₃-alkoxy, carboxy, C₁₋₃-alkoxycarbonyl, aminocarbonyl, C₁₋₄-alkylamino-carbonyl, di-(C₁₋₄-alkyl)-amino-carbonyl, aminosulphonyl, C₁₋₃-alkyl-aminosulphonyl, di-(C₁₋₃-alkyl)-aminosulphonyl, C₁₋₃-alkyl-sulphonylamino, nitro or cyano groups, while the substituents may be identical or different, or two adjacent hydrogen atoms of the phenyl groups may be replaced by a methylenedioxy group,
and
R₅ denotes a hydrogen atom or a C₁₋₃-alkyl group,
while by the term aryl group is meant a phenyl or naphthyl group optionally mono- or disubstituted by a fluorine, chlorine, bromine or iodine atom, by a cyano, trifluoromethyl, nitro, carboxy, aminocarbonyl, C₁₋₃-alkyl or C₁₋₃-alkoxy group and
by the term heteroaryl group is meant a monocyclic 5- or 6-membered heteroaryl group optionally substituted in the carbon skeleton by a C₁₋₃-alkyl group, wherein
the 6-membered heteroaryl group contains one, two or three nitrogen atoms and
the 5-membered heteroaryl group contains an imino group optionally substituted by a C₁₋₃-alkyl or phenyl-C₁₋₃-alkyl group, an oxygen or sulphur atom or
an imino group optionally substituted by a C₁₋₃-alkyl or phenyl-C₁₋₃-alkyl group or an oxygen or sulphur atom and additionally a nitrogen atom or
an imino group optionally substituted by a C₁₋₃-alkyl or phenyl-C₁₋₃-alkyl group and two nitrogen atoms,
and moreover a phenyl ring may be fused to the abovementioned monocyclic heterocyclic groups via two adjacent carbon atoms and the bond is via a nitrogen atom or via a carbon atom of the heterocyclic moiety of a fused phenyl ring,
the hydrogen atoms in the abovementioned alkyl and alkoxy groups or in the alkyl moieties contained in the above-defined groups of formula I may be wholly or partly replaced by fluorine atoms,
the saturated, alkyl and alkoxy moieties containing more than 2 carbon atoms present in the groups defined above also include the branched isomers thereof such as for example the isopropyl, tert.butyl, isobutyl group, unless otherwise stated, and
wherein additionally the hydrogen atom of any carboxy group present or a hydrogen atom bound to a nitrogen atom, for example an amino, alkylamino or imino group or a saturated N-heterocycle such as the piperidinyl group, may be replaced in each case by a group which can be cleaved *in vivo*,
the tautomers, the diastereomers, the enantiomers, the mixtures thereof and the salts thereof,
for the preparation of a medicament for the prevention or treatment of a fibrotic disease selected from the group consisting of fibrosis and remodeling of lung tissue in chronic obstructive pulmonary disease, fibrosis and remodeling of lung tissue in chronic bronchitis, fibrosis and remodeling of lung tissue in emphysema, lung fibrosis and pulmonary diseases with a fibrotic component, fibrosis and remodeling in asthma.

2. Use in accordance with claim 1 wherein the substituted indolinone of general formula I is selected from the group consisting of:
(a) methyl 3-(Z)-[1-{4-[N-(2-dimethylamino-ethyl)-N-methylcarbamoyl]-phenylamino)-1-phenyl-methylidene]-2-indolinone-6-carboxylate
(b) methyl 3-(Z)-[1-{4-[N-(3-dimethylamino-propyl)-N-methyl-carbamoyl]-phenylamino}-1-phenyl-methylidene]-2-indolinone-6-carboxylate
(c) methyl 3-(Z)-[1-{4-[(4-methyl-piperazin-1-yl)-carbonyl]-phenylamino}-1-phenyl-methylidene]-2-indolinone-6-carboxylate
(d) methyl 3-(Z)-[1-{4-[(4-hydroxy-piperidin-1-yl)-carbonyl]-phenylamino}-1-phenyl-methylidene]-2-indolinone-6-carboxylate
(e) methyl 3-(Z)-[1-{4-[(piperazin-1-yl)-carbonyl]-phenylamino}-1-phenyl-methylidene]-2-indolinone-6-carboxylate
(f) methyl 3-(Z)-[1-{4-[N-(2-methylamino-ethyl)-N-methyl-carbamoyl]-phenylamino}-1-phenyl-methylidene]-2-indolinone-6-carboxylate
(g) methyl 3-(Z)-[1-{4-[(4-dimetylamino-piperidin-1-yl)-carbonyl]-phenylamino}-1-phenyl-methyldiene]-2-indolinone-6-carboxylate
(h) methyl 3-(Z)-[1-{4-[(4-ethyl-piperazin-1-yl)-carbonyl]-phenylamino}-1-phenyl-methyldiene]-2-indolinone-6-carboxylate
(i) methyl 3-(Z)-[1-{4-[(4-(2-hydroxy-ethyl)-piperazin-1-yl)-carbonyl]-phenylamino}-1-phenyl-methyldiene]-2-indolinone-6-carboxylate
(k) methyl-3-(Z)-{1-[4-(5-methyl-2,5-diazabicyclo[2.2.1] hept-2-yl-carbonyl)-phenylamino]-1-phenyl-methyldiene}-2-indolinone-6-carboxylate
(l) (*S*)-3-(Z)-[1-{4-[(3,4-dimethyl-piperazin-2-yl)-carbonyl]-phenylamino}-1-phenyl-methylidene] -6-methoxycarbonyl-2-indolinone, and
(m) 3-(Z)-(1-{5-[(2-dimethylamino-ethyl)-N-methylcarbamoyl]-furan-2-yl-amino}-1-phenyl-methylidene] -6-methoxycarbonyl-2-indolinone,
the tautomers, the diastereomers, the enantiomers, the mixtures thereof and the salts thereof.

3. Use in accordance with any one of claims 1 or 2 wherein the disease is selected from the group consisting of the lung fibrosis and pulmonary diseases with a fibrotic component selected from idiopathic pulmonary fibrosis, giant cell interstitial pneumonia, sarcodosis, cystic fibrosis, respiratory distress syndrome, drug-induced lung fibrosis, granulomatosis, silicosis, asbestosis, systemic scleroderma.

4. Use in accordance with any one of claims 1 to 3 wherein the disease is idiopathic pulmonary fibrosis.

5. Use in accordance with any one of claims 1 to 4 wherein the treatment is a combined treatment with a further pharmacologically active substance selected from the group consisting of anticholinergic agents, beta-2 mimetics, steroids, PDE-IV.inhibitors, p38 MAP kinase inhibitors, NK₁ antagonists, LTD4 antagonists, EGFR inhibitors and endothelin-antagonists.

6. A pharmaceutical composition comprising a substituted indolinone of formula I as defined in any one of claims 1 or 2, optionally together with one or more pharmaceutically acceptable carriers or excipients, for the prevention or treatment of a fibrotic disease selected from the group consisting of fibrosis and remodeling of lung tissue in chronic obstructive pulmonary disease, fibrosis and remodeling of lung tissue in chronic bronchitis, fibrosis and remodeling of lung tissue in emphysema, lung fibrosis and pulmonary diseases with a fibrotic component, fibrosis and remodeling in asthma, radiation-induced fibrosis.

7. The pharmaceutical composition in accordance with claim 6, wherein the disease is selected from the group consisting of the lung fibrosis and pulmonary diseases with a fibrotic component selected from idiopathic pulmonary fibrosis, giant cell interstitial pneumonia, sarcodosis, cystic fibrosis, respiratory distress syndrome, drug-induced lung fibrosis, granulomatosis, silicosis, asbestosis, systemic scleroderma.

8. The pharmaceutical composition in accordance with claim 7 wherein the disease is idiopathic pulmonary fibrosis.

9. The pharmaceutical composition in accordance with any one of claims 6 to 8, wherein the treatment is a combined treatment with a further pharmacologically active substance selected from the group consisting of anticholinergic agents, beta-2 mimetics, steroids, PDE-IV inhibitors, p38 MAP kinase inhibitors, NK₁ antagonists, LTD4 antagonists, EGFR inhibitors and endothelin-antagonists.said further pharmacologically active substance being administered either simultaneously or sequentially.

10. Pharmaceutical composition containing a substituted indolinone of formula I as defined in any one of claims 1 or 2 in combination with a further pharmacologically active substance selected from the group consisting of anticholinergic agents, beta-2 mimetics, steroids, PDE-IV inhibitors, p38 MAP kinase inhibitors, NK₁ antagonists, LTD4 antagonists, EGFR inhibitors and endothelin-antagonists, optionally together with one or more pharmaceutically acceptable carriers or excipients.

## Patentansprüche

1. Verwendung der Indolinone der allgemeinen Formel 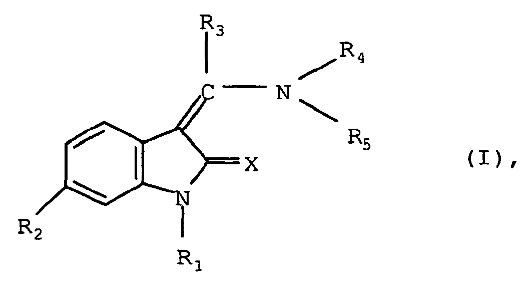 substituiert in der 6-Position, worin
X ein Sauerstoff- oder Schwefelatom bezeichnet,
R₁ ein Wasserstoffatom oder eine Propharmakongruppe bezeichnet,
R₂ eine Carboxygruppe, eine geradkettige oder verzweigte C₁₋₆-Alkoxycarbonylgruppe, eine C₄₋₇-Cycloalkoxycarbonyl- oder eine Aryloxycarbonylgruppe bezeichnet,
R₃ ein Wasserstoffatom, eine C₁₋₆-Alkyl, C₃₋₇-Cycloalkyl-, Trifluormethyl- oder Heteroarylgruppe bezeichnet,
eine Phenyl- oder Naphthylgruppe darstellt, oder eine Phenyl- oder Naphthylgruppe, die mono- oder disubstituiert ist mit einem Fluor-, Chlor-, Brom- oder Iodatom, mit einer Trifluormethyl-, C₁₋₃-Alkyl- oder C₁₋₃-Alkoxygruppe, wobei im Falle der Disubstitution die Substituenten identisch oder verschieden sein können,
R₄ eine Phenyl-, Pyrrolyl- oder Furanylgruppe bezeichnet, substituiert mit der Gruppe R₆, die zusätzlich mono- oder disubstituiert sein kann mit Fluor-, Chlor-, Brom- oder Iodatomen, mit C₁₋₅-Alkyl-, Trifluormethyl-, Hydroxy-, C₁₋₃-Alkoxy-, Carboxy-, C₁₋₃-Alkoxycarbonyl-, Amino-, Acetylamino-, C₁₋₃-Alkylsulfonyl-amino-, Aminocarbonyl-, C₁₋₃-Alkylaminocarbonyl, Di-(C₁₋₃-alkyl)amino-carbonyl-, Aminosulfonyl-, C₁₋₃-Alkylaminosulfonyl-, Di-(C₁₋₃-alkyl)aminosulfonyl-, Nitro- oder Cyanogruppen, wobei die Substituenten identisch oder verschieden sein können, und worin
R₆ eine Aminocarbonyl-, C₁₋₄-Alkylamioncarbonyl-, N-(C₁₋₅-Alkyl)-C₁₋₃-alkyl-aminocarbonyl-, C₃₋₇-Cycloalkylaminocarbonyl-, N-(C₁₋₅-Alkyl)-C₃₋₇-cycloalkyl-aminocarbonyl-, (Phenyl-C₁₋₃-alkyl)aminocarbonyl-, N-(C₁₋₃-Alkyl)-phenyl-C₁₋₃-alkylaminocarbonylgruppe, eine C₁₋₃-Alkylamioncarbonyl- oder N-(C₁₋₃-Alkyl)-C₁₋₃-alkylaminocarbonylgruppe bezeichnet, worin ein oder zwei Alkylreste unabhängig voneinander durch eine Nitro-, Cyano-, Carbamoyl-, N-(C₁₋₃-Alkyl)carbamoyl-, Di-N-(C₁₋₃-alkyl)carbamoyl-, Carboxy- oder C₁₋₃-Alkoxycarbonylgruppe substituiert sind, oder sie sind in der 2- oder 3-Position mit einer Amino-, (C₁₋₃-Alkyl)amino-, Di-(C₁₋₃-alkyl)amino-, (C₁₋₄-Alkoxycarbonyl)-amino-, N-(C₁₋₄-Alkoxycarbonyl)-N-(C₁₋₃-alkyl)amino-, Piperazino-, N-(C₁₋₃-alkyl)piperazino-, einer 4- bis 7-gliedrigen Cycloalkyleniminogruppe, einer Hydroxy- oder Methoxygruppe, einer 4- bis 7-gliedrigen Cycloalkyleniminocarbonylgruppe substituiert, worin
der Cycloalkylenrest über zwei benachbarte Ringatome mit einem Phenylring kondensiert sein kann, oder mit einer Methylen- oder Ethylengruppe über zwei nicht benachbarte Ringatome eine Brücke bilden kann, oder
ein oder zwei Wasserstoffatome können jeweils durch eine C₁₋₃-Alkylgruppe ersetzt sein und/oder
in jedem Fall kann die Methylengruppe in der 4-Position einer 6- oder 7-gliedrigen Cycloalkyleniminocarbonylgruppe substituiert sein mit einer Carboxy-, C₁₋₄-Alkoxycarbonyl-, Aminocarbonyl-, C₁₋₃-Alkylaminocarbonyl-, Di(C₁₋₃-alkyl)aminocarbonyl-, Di(C₁₋₃-alkyl)amino-C₁₋₃-alkyl-, Di(C₁₋₃-alkyl)amino-, Phenyl-C₁₋₃-alkylamino- oder N-(C₁₋₃-Alkyl)phenyl-C₁₋₃-alkylaminogruppe oder einer Hydroxy- oder Methoxygruppe oder
durch ein Sauerstoff- oder Schwefelatom, eine Sulfinyl-, Sulfonyl- oder -NH-Gruppe oder durch ein Stickstoffatom, das substituiert ist mit einer C₁₋₃-Alkyl-, Phenyl-, C₁₋₃-Alkylcarbonyl-, C₁₋₄-Alkoxycarbonyl-, Di(C₁₋₃-alkyl)amino-C₁₋₃-alkyl-, Hydroxy-C₂₋₃-alkyl- oder Benzoylgruppe ersetzt sein,
wobei sämtliche der einfach gebundenen oder kondensierten Phenylgruppen, enthalten in den bei R₆ erwähnten Gruppen, mono- oder disubstituiert sein können mit Fluor-, Chlor-, Brom- oder Iodatomen, mit C₁₋₅-Alkyl-, Trifluormethyl-, Hydroxy-, C₁₋₃-Alkoxy-, Carboxy-, C₁₋₃-Alkoxycarbonyl-, Aminocarbonyl-, C₁₋₄-Alkylaminocarbonyl-, Di(C₁₋₄-alkyl)aminocarbonyl-, Aminosulfonyl-, C₁₋₃-Alkylaminosulfonyl-, Di(C₁₋₃-alkyl)aminosulfonyl-, C₁₋₃-Alkylsulfonylamino-, Nitro- oder Cyanogruppen, wobei die Substituenten identisch oder verschieden sein können, oder zwei benachbarte Wasserstoffatome der Phenylgruppen können durch eine Methylendioxygruppe ersetzt sein,
und
R₅ bezeichnet ein Wasserstoffatom oder eine C₁₋₃-Alkylgruppe,
während mit dem Begriff Arylgruppe eine Phenyl- oder Naphthylgruppe gemeint ist, gegebenenfalls mono- oder disubstituiert mit einem Fluor-, Chlor-, Brom-oder Iodatom, mit einer Cyano-, Trifluormethyl-, Nitro-, Carboxy-, Aminocarbonyl-, C₁₋₃-Alkyl- oder C₁₋₃-Alkoxygruppe, und
mit dem Begriff Heteroarylgruppe ist eine monocyclische 5- oder 6-gliedrige Heteroarylgruppe gemeint, gegebenenfalls substituiert im Kohlenstoffskelett mit einer C₁₋₃-Alkylgruppe, worin
die 6-gliedrige Heteroarylgruppe ein, zwei oder drei Stickstoffatome enthält, und die 5-gliedrige Heteroarylgruppe eine Iminogruppe enthält, gegebenenfalls substituiert mit einer C₁₋₃-Alkyl- oder Phenyl-C₁₋₃-alkylgruppe, einem Sauerstoff-oder Schwefelatom oder einer Iminogruppe, gegebenenfalls substituiert mit einer C₁₋₃-Alkyl- oder Phenyl-C₁₋₃-alkylgruppe oder einem Sauerstoff- oder Schwefelatom und zusätzlich einem Stickstoffatom, oder
einer Iminogruppe, gegebenenfalls substituiert mit einer C₁₋₃-Alkyl- oder Phenyl-C₁₋₃-alkylgruppe und zwei Stickstoffatomen,
und darüber hinaus kann ein Phenylring an die zuvor erwähnten monocyclischen heterocyclischen Gruppen über zwei benachbarte Kohlenstoffatome kondensiert sein und die Bindung erfolgt über ein Stickstoffatom oder über ein Kohlenstoff atom des heterocyclischen Rests eines kondensierten Phenylrings,
wobei die Wasserstoffatome in den zuvor erwähnten Alkyl- und Alkoxygruppen oder in den Alkylresten, die in den oben definierten Gruppen der Formel I enthalten sind, vollständig oder teilweise durch Fluoratome ersetzt sein können,
wobei die gesättigten Alkyl- und Alkoxyreste, die mehr als 2 Kohlenstoffatome enthalten, die in den oben definierten Gruppen vorliegen, ebenfalls die verzweigten Isomere hiervon umfassen, wie beispielsweise die Isopropyl-, tert-Butyl-, Isobutylgruppe, sofern nicht anders angegeben, und
worin zusätzlich das Wasserstoffatom irgendeiner vorliegenden Carboxygruppe oder ein Wasserstoffatom, gebunden an ein Stickstoffatom, beispielsweise einer Amino-, Alkylamino- oder Iminogruppe, oder ein gesättigter N-Heterocyclus, wie die Piperidinylgruppe, in jedem Fall durch eine Gruppe ersetzt sein kann, die *in* vivo gespalten werden kann,
die Tautomeren, die Diastereomeren, die Enantiomeren, die Mischungen hiervon und die Salze hiervon,
zur Herstellung eines Arzneimittels zur Vorbeugung oder Behandlung einer fibrotischen Erkrankung, ausgewählt aus der Gruppe, bestehend aus Fibrose und
Umbildung von Lungengewebe bei chronisch obstruktiver pulmonaler Erkrankung, Fibrose und Umbildung von Lungengewebe bei chronischer Bronchitis, Fibrose und Umbildung von Lungengewebe bei einem Emphysem, Lungenfibrose und pulmonale Erkrankungen mit einer fibrotischen Komponente, Fibrose und Umbildung bei Asthma.

2. Verwendung nach Anspruch 1, wobei das substituierte Indolinon der allgemeinen Formel I ausgewählt ist aus der Gruppe, bestehend aus:
(a) Methyl-3-(Z)-[1-{4-[N-(2-dimethylaminoethyl)-N-methylcarbamoyl]-phenyl-amino}-1-phenylmethyliden]-2-indolinon-6-carboxylat,
(b) Methyl-3-(Z)-[1-{4-[N-(3-dimethylaminopropyl)-N-methylcarbamoyl]-phenylamino}-1-phenylmethyliden]-2-indolinon-6-carboxylat,
(c) Methyl 3-(Z)-[1-{4-[(4-methyl-piperazin-1-yl)-carbonyl]-phenylamino}-1-phenyl-methyliden]-2-indolinon-6-carboxylat,
(d) Methyl 3-(Z)-[1-{4-[(4-hydroxy-piperidin-1-yl)-carbonyl]-phenylamino}-1-phenyl-methyliden]-2-indolinon-6-carboxylat,
(e) Methyl 3-(Z)-[1-{4-[(piperazin-1-yl)-carbonyl]-phenylamino}-1-phenyl-methyliden]-2-indolinon-6-carboxylat,
(f) Methyl 3-(Z)-[1-{4-[N-(2-methylamino-ethyl)-N-methyl-carbamoyl]-phenylamino}-1-phenyl-methyliden]-2-indolinon-6-carboxylat,
(g) Methyl 3-(Z)-[1-{4-[(4-dimetylamino-piperidin-1-yl)-carbonyl]-phenylamino}-1-phenyl-methyldien]-2-indolinon-6-carboxylat,
(h) Methyl 3-(Z)-[1-{4-[(4-ethyl-piperazin-1-yl)-carbonyl]-phenylamino}-1-phenyl-methyldien]-2-indolinon-6-carboxylat,
(i) Methyl 3-(Z)-[1-{4-[(4-(2-hydroxy-ethyl)-piperazin-1-yl)-carbonyl]-phenylamino}-1-phenyl-methyldien]-2-indolinon-6-carboxylat,
(k) Methyl-3-(Z)-{1-[4-(5-methyl-2,5-diazabicyclo[2.2.1] hept-2-yl-carbonyl)-phenylamino]-1-phenyl-methyldien}-2-indolinon-6-carboxylat,
(l) (S)-3-(Z)-[1-{4-[(3,4-Dimethylpiperazin-1-yl)carbonyl]phenylamino}-1-phenylmethyliden]-6-methoxycarbonyl-2-indolinon und
(m) 3-(Z)-[1-{5-[(2-Dimethylaminoethyl)-N-methylcarbamoyl]furan-2-yl-amino}-1-phenylmethyliden]-6-methoycarbonyl-2-indolinon, die Tautomeren, die Diastereomeren, die Enantiomeren, die Mischungen hiervon und die Salze hiervon.

3. Verwendung nach irgendeinem der Ansprüche 1 oder 2, wobei die Krankheit, ausgewählt ist aus der Gruppe, bestehend aus Lungenfibrose und pulmonalen Erkrankungen mit einer fibrotischen Komponente, ausgewählt aus idiopathischer pulmonaler Fibrose, interstitieller Riesenzellenpneumonie, Sarcodose, cystischer Fibrose, respiratory distress-Syndrom, Arzneimittel-induzierter Lungenfibrose, Granulomatose, Silicose, Asbestose, systemischer Sklerodermie.

4. Verwendung nach irgendeinem der Ansprüche 1 bis 3, wobei die Erkrankung idiopathische pulmonale Fibrose darstellt.

5. Verwendung nach irgendeinem der Ansprüche 1 bis 4, wobei die Behandlung eine kombinierte Behandlung mit einer weiteren pharmakologisch aktiven Substanz darstellt, ausgewählt aus der Gruppe, bestehend aus anticholinergen Mitteln, β-2-Mimetika, Steroiden, PDE-IV-Inhibitoren, p38-MAP-Kinase-Inhibitoren, NK₁-Antagonisten, LTD4-Antagonisten, EGFR-Inhibitoren und Endothelin-Antagonisten.

6. Pharmazeutische Zusammensetzung, umfassend ein substituiertes Indolinon der Formel I, wie in irgendeinem der Ansprüche 1 oder 2 definiert, gegebenenfalls zusammen mit einem oder mehreren pharmazeutisch akzeptablen Trägern oder Hilfsstoffen zur Vorbeugung oder Behandlung einer fibrotischen Erkrankung, ausgewählt aus der Gruppe, bestehend aus Fibrose und Umbildung von Lungengewebe bei chronisch obstruktiver pulmonaler Erkrankung, Fibrose und Umbildung von Lungengewebe bei chronischer Bronchitis, Fibrose und Umbildung von Lungengewebe bei einem Emphysem, Lungenfibrose und pulmonale Erkrankungen mit einer fibrotischen Komponente, Fibrose und Umbildung bei Asthma, strahlungsinduzierter Fibrose.

7. Pharmazeutische Zusammensetzung nach Anspruch 6, wobei die Krankheit ausgewählt ist aus der Gruppe, bestehend aus Lungenfibrose und pulmonalen Erkrankungen mit einer fibrotischen Komponente, ausgewählt aus idiopathischer pulmonaler Fibrose, interstitieller Riesenzellenpneumonie, Sarcodose, cystischer Fibrose, respiratory distress-Syndrom, Arzneimittel-induzierter Lungenfibrose, Granulomatose, Silicose, Asbestose, systemischer Sklerodermie.

8. Pharmazeutische Zusammensetzung nach Anspruch 7, wobei die Erkrankung idiopathische pulmonale Fibrose darstellt.

9. Pharmazeutische Zusammensetzung nach irgendeinem der Ansprüche 6 bis 8,
wobei die Behandlung eine kombinierte Behandlung mit einer weiteren pharmakologisch aktiven Substanz darstellt, ausgewählt aus der Gruppe, bestehend aus anticholinergen Mitteln, β-2-Mimetika, Steroiden, PDE-IV-Inhibitoren, p38-MAP-Kinase-Inhibitoren, NK₁-Antagonisten, LTD4-Antagonisten, EGFR-Inhibitoren und Endothelin-Antagonisten, wobei eine weitere pharmakologisch aktive Substanz entweder simultan oder darauf folgend verabreicht wird.

10. Pharmazeutische Zusammensetzung, enthaltend ein substituiertes Indolinon der Formel I, wie in irgendeinem der Ansprüche 1 oder 2 definiert, in Kombination mit einer weiteren pharmakologisch aktiven Substanz, ausgewhält aus der Gruppe, bestehend aus anticholinergen Mitteln, β-2-Mimetika, Steroiden, PDE-IV-Inhibitoren, p38-MAP-Kinase-Inhibitoren, NK₁-Antagonisten, LTD4-Antagonisten, EGFR-Inhibitoren und Endothelin-Antagonisten, gegebenenfalls zusammen mit einem oder mehreren pharmakologisch akzeptablen Trägem oder Hilfsstoffen.

## Revendications

1. Utilisation des indolinones de formule générale 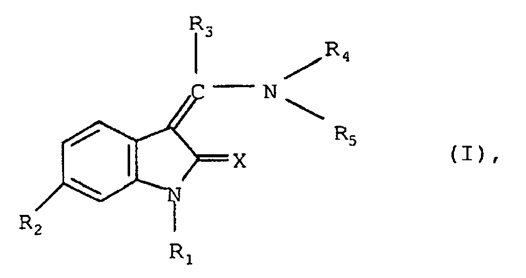 substituées à la position 6, où
X désigne un atome d'oxygène ou de soufre,
R₁ désigne un atome d'hydrogène ou un groupe de promédicament,
R₂ désigne un groupe carboxy, un groupe C₁₋₆-alcoxycarbonyle linéaire ou ramifié, un groupe C₄₋₇-cycloalcoxycarbonyle ou un groupe aryloxycarbonyle,
R₃ désigne un atome d'hydrogène, un groupe C₁₋₆-alkyle, C₃₋₇-cycloalkyle, trifluorométhyle ou hétéroaryle,
un groupe phényle ou naphtyle, ou un groupe phényle ou naphtyle mono- ou disubstitué par un atome de fluor, de chlore, de brome ou d'iode, par un groupe trifluorométhyle, C₁₋₃-alkyle ou C₁₋₃-alcoxy, tandis que, en cas de disubstitution, les substituants peuvent être identiques ou différents,
R₄ désigne un groupe phényle, pyrrolyle ou furanyle substitué par le groupe R₆, qui peut en outre être mono- ou disubstitué par des atomes de fluor, de chlore, de brome ou d'iode, par des groupes C₁₋₅-alkyle, trifluorométhyle, hydroxy, C₁₋₃-alcoxy, carboxy, C₁₋₃-alcoxycarbonyle, amino, acétylamino, C₁₋₃-alkyl-sulfonylamino, aminocarbonyle, C₁₋₃-alkyl-aminocarbonyle, di-(C₁₋₃-alkyl)-aminocarbonyle, aminosulfonyle, C₁₋₃-alkyl-aminosulfonyle, di-(C₁₋₃-alkyl)-aminosulfonyle, nitro ou cyano, tandis que les substituants peuvent être identiques ou différents et où
R₆ désigne un groupe aminocarbonyle, C₁₋₄-alkylaminocarbonyle, N-(C₁₋₅-alkyl)-C₁₋₃-alkylaminocarbonyle, C₃₋₇-cycloalkyl-aminocarbonyle, N-(C₁₋₅-alkyl)-C₃₋₇-cycloalkylaminocarbonyle, (phényl-C₁₋₃-alkyl)amino-carbonyle, N-(C₁₋₃-alkyl)-phényl-C₁₋₃-alkylamino-carbonyle,
un groupe C₁₋₃-alkylaminocarbonyle ou N-(C₁₋₃-alkyl)-C₁₋₃-alkylaminocarbonyle où un ou deux groupements alkyle sont substitués indépendamment l'un de l'autre par un groupe nitro, cyano, carbamoyle, N-(C₁₋₃-alkyl)-carbamoyle, di-N-(C₁₋₃-alkyl)-carbamoyle, carboxy ou C₁₋₃-alcoxycarbonyle ou sont substitués à la position 2 ou 3 par un groupe amino, (C₁₋₃-alkyl)-amino, di-(C₁₋₃-alkyl)-amino, (C₁₋₄-alcoxycarbonyl)-amino, N-(C₁₋₄-alcoxycarbonyl)-N-(C₁₋₃-alkyl)-amino, pipérazino, N-(C₁₋₃-alkyl)-pipérazino, un groupe cycloalkylèneimino à 4 à 7 chaînons, un groupe hydroxy ou méthoxy,
un groupe cycloalkylèneiminocarbonyle à 4 à 7 chaînons où
le groupement cycloalkylène peut être condensé à un cycle phényle via deux atomes du cycle adjacents ou peut former un pont avec un groupe méthylène ou éthylène via deux atomes du cycle non adjacents ou
un ou deux atomes d'hydrogène peuvent être remplacés chacun par un groupe C₁₋₃-alkyle et/ou
dans chaque cas le groupe méthylène à la position 4 d'un groupe cycloalkylèneiminocarbonyle à 6 ou 7 chaînons peut être substitué par un groupe carboxy, C₁₋₄-alcoxycarbonyle, aminocarbonyle, C₁₋₃-alkylaminocarbonyle, di-(C₁₋₃-alkyl)-aminocarbonyle, di-(C₁₋₃-alkyl)-amino-C₁₋₃-alkyle, di-(C₁₋₃-alkyl)-amino, phényl-C₁₋₃-alkylamino ou N-(C₁₋₃-alkyl)-phényl-C₁₋₃-alkylamino ou un groupe hydroxy ou méthoxy ou
peut être remplacé par un atome d'oxygène ou de soufre, par un groupe sulfinyle, sulfonyle ou -NH ou par un atome d'azote qui est substitué par un groupe C₁₋₃-alkyle, phényle, C₁₋₃-alkyl-carbonyle, C₁₋₄-alcoxycarbonyle, di-(C₁₋₃-alkyl)-amino-C₁₋₃-alkyle, hydroxy-C₂₋₃-alkyle ou benzoyle,
tandis que tous les groupes phényle à simple liaison ou condensés contenus dans les groupes mentionnés sous R₆ peuvent être mono- ou disubstitués par des atomes de fluor, de chlore, de brome ou d'iode, par des groupes C₁₋₅-alkyle, trifluorométhyle, hydroxy, C₁₋₃-alcoxy, carboxy, C₁₋₃-alcoxycarbonyle, aminocarbonyle, C₁₋₄-alkylamino-carbonyle, di-(C₁₋₄-alkyl)-amino-carbonyle, aminosulfonyle, C₁₋₃-alkyl-aminosulfonyle, di-(C₁₋₃ -alkyl)-aminosulfonyle, C₁₋₃-alkyl-sulfonylamino, nitro ou cyano, tandis que les substituants peuvent être identiques ou différents, ou deux atomes d'hydrogène adjacents des groupes phényle peuvent être remplacés par un groupe méthylènedioxy,
et
R₅ désigne un atome d'hydrogène ou un groupe C₁₋₃-alkyle,
tandis que, par le terme aryle on entend un groupe phényle ou naphtyle éventuellement mono- ou disubstitué par un atome de fluor, de chlore, de brome ou d'iode, par un groupe cyano, trifluorométhyle, nitro, carboxy, aminocarbonyle, C₁₋₃-alkyle ou C₁₋₃-alcoxy et
par le terme groupe hétéroaryle on entend un groupe hétéroaryle à 5 ou 6 chaînons monocyclique éventuellement substitué dans le squelette carboné par un groupe C₁₋₃-alkyle, où
le groupe hétéroaryle à 6 chaînons contient un, deux ou trois atomes d'azote et
le groupe hétéroaryle à 5 chaînons contient un groupe imino éventuellement substitué par un groupe C₁₋₃-alkyle ou phényl-C₁₋₃-alkyle, un atome d'oxygène ou de soufre ou un groupe imino éventuellement substitué par un groupe C₁₋₃-alkyle ou phényl-C₁₋₃-alkyle ou un atome d'oxygène ou de soufre et en outre un atome d'azote ou
un groupe imino éventuellement substitué par un groupe C₁₋₃-alkyle ou phényl-C₁₋₃-alkyle et deux atomes d'azote,
et en outre un cycle phényle peut être condensé aux groupes hétérocycliques monocycliques mentionnés ci-dessus via deux atomes de carbone adjacents et la liaison est via un atome d'azote ou via un atome de carbone du groupement hétérocyclique d'un cycle phényle condensé,
les atomes d'hydrogène dans les groupes alkyle et alcoxy mentionnés précédemment ou dans les groupements alkyle contenus dans les groupes de la formule I définis précédemment peuvent être totalement ou partiellement remplacés par des atomes de fluor,
les groupements alkyle et alcoxy saturés contenant plus de 2 atomes de carbone présents dans les groupes définis ci-dessus incluent aussi leurs isomères ramifiés comme, par exemple, le groupe isopropyle, tert.butyle, isobutyle, sauf indication contraire, et
où en outre l'atome d'hydrogène d'un groupe carboxy quelconque présent ou un atome d'hydrogène lié à un atome d'azote, par exemple un groupe amino, alkylamino ou imino ou un N-hétérocycle saturé comme le groupe pipéridinyle, peut être remplacé dans chaque cas par un groupe qui peut être clivé *in vivo,*
de leurs tautomères, de leurs diastéréoisomères, de leurs énantiomères, de leurs mélanges et de leurs sels,
pour la préparation d'un médicament pour la prévention ou le traitement d'une maladie fibrotique choisie dans le groupe consistant en la fibrose et le remodelage du tissu pulmonaire dans la bronchopneumopathie obstructive chronique, la fibrose et le remodelage du tissu pulmonaire dans la bronchite chronique, la fibrose et le remodelage du tissu pulmonaire dans l'emphysème, la fibrose pulmonaire et les maladies pulmonaires à composante fibrotique, la fibrose et le remodelage dans l'asthme.

2. Utilisation selon la revendication 1 où l'indolinone substituée de formule générale 1 est choisie dans le groupe consistant en :
(a) le 3-(Z)-[1-{4-[N-(2-diméthylamino-éthyl)-N-méthyl-carbamoyl]-phénylamino}-1-phényl-méthylidène]-2-indolinone-6-carboxylate de méthyle
(b) le 3-(Z)-[1-{4-[N-(3-diméthylamino-propyl)-N-méthyl-carbamoyl]-phénylamino}-1-phényl-méthylidène]-2-indolinone-6-carboxylate de méthyle
(c) le 3-(Z)-[1-{4-[(4-méthyl-pipérazin-1-yl)-carbonyl]-phénylamino}-1-phényl-méthylidène]-2-indolinone-6-carboxylate de méthyle
(d) le 3-(Z)-[1-{4-[(4-hydroxy-pipéridin-1-yl)-carbonyl]-phénylamino}-1-phényl-méthylidene]-2-indolinone-6-carboxylate de méthyle
(e) le 3-(Z)-[1-{4-[(pipérazin-1-yl)-carbonyl]-phénylamino}-1-phényl-méthylidène]-2-indolinone-6-carboxylate de méthyle
(f) le 3-(Z)-[1-{4-[N-(2-méthylamino-éthyl)-N-méthyl-carbamoyl]-phénylamino}-1-phényl-méthylidène]-2-indolinone-6-carboxylate de méthyle
(g) le 3-(Z)-[1-{4-[(4-dimétylamino-pipéridin-1-yl)-carbonyl]-phénylamino}-1-phényl-méthyldiène]-2-indolinone-6-carboxylate de méthyle
(h) le 3-(Z)-[1-{4-[(4-éthyl-pipérazin-1-yl)-carbonyl]-phénylamino}-1-phényl-méthyldiène]-2-indolinone-6-carboxylate de méthyle
(i) le 3-(Z)-[1-{4-[(4-(2-hydroxy-éthyl)-pipérazin-1-yl)-carbonyl]-phénylamino}-1-phényl-méthyldiène]-2-indolinone-6-carboxylate de méthyle
(k) le 3-(Z)-{1-[4-(5-méthyl-2,5-diazabicyclo[2.2.1]hept-2-yl-carbonyl)-phénylamino]-1-phényl-méthyldiène}-2-indolinone-6-carboxylate de méthyle
(l) la (*S*)-3-(Z)-[1-(4-[(3,4-diméthyl-pipérazin-1-yl)-carbonyl]-phénylamino)-1-phényl-méthylidène]-6-méthoxycarbonyl-2-indolinone et
(m) la 3-(Z)-[1-{5-[(2-diméthylamino-éthyl)-N-méthyl-carbamoyl]-furan-2-yl-amino}-1-phényl-méthylidène]-6-méthoxycarbonyl-2-indolinone, leurs tautomères, leurs diastéréoisomères, leurs énantiomères, leurs mélanges et leurs sels.

3. Utilisation selon l'une quelconque des revendications 1 ou 2 où la maladie est choisie dans le groupe consistant en la fibrose pulmonaire et les maladies pulmonaires à composante fibrotique choisies parmi la fibrose pulmonaire idiopathique, la pneumonie interstitielle à cellules géantes, la sarcoïdose, la mucoviscidose, le syndrome de détresse respiratoire, la fibrose pulmonaire induite par des médicaments, la granulomatose, la silicose, l'asbestose, la sclérodermie systémique.

4. Utilisation selon l'une quelconque des revendications 1 à 3 où la maladie est la fibrose pulmonaire idiopathique.

5. Utilisation selon l'une quelconque des revendications 1 à 4 où le traitement est un traitement combiné avec une autre substance pharmacologiquement active choisie dans le groupe consistant en les agents anticholinergiques, les bêta-2 mimétiques, les stéroïdes, les inhibiteurs de PDE-IV, les inhibiteurs de MAP kinase p38, les antagonistes de NK₁, les antagonistes de LTD4, les inhibiteurs de EGFR et les antagonistes d'endothéline.

6. Composition pharmaceutique comprenant une indolinone substituée de formule 1 selon l'une quelconque des revendications 1 ou 2 éventuellement en même temps qu'un ou plusieurs vecteurs ou excipients pharmaceutiquement acceptables pour la prévention ou le traitement d'une maladie fibrotique choisie dans le groupe consistant en la fibrose et le remodelage du tissu pulmonaire dans la bronchopneumopathie obstructive chronique, la fibrose et le remodelage du tissu pulmonaire dans la bronchite chronique, la fibrose et le remodelage du tissu pulmonaire dans l'emphysème, la fibrose pulmonaire et les maladies pulmonaires à composante fibrotique, la fibrose et le remodelage dans l'asthme, la fibrose induite par des rayonnements.

7. Composition pharmaceutique selon la revendication 6 où la maladie est choisie dans le groupe consistant en la fibrose pulmonaire et les maladies pulmonaires à composante fibrotique choisies parmi la fibrose pulmonaire idiopathique, la pneumonie interstitielle à cellules géantes, la sarcoïdose, la mucoviscidose, le syndrome de détresse respiratoire, la fibrose pulmonaire induite par des médicaments, la granulomatose, la silicose, l'asbestose, la sclérodermie systémique.

8. Composition pharmaceutique selon la revendication 7 où la maladie est la fibrose pulmonaire idiopathique.

9. Composition pharmaceutique selon l'une quelconque des revendications 6 à 8 où le traitement est un traitement combiné avec une autre substance pharmacologiquement active choisie dans le groupe consistant en les agents anticholinergiques, les bêta-2 mimétiques, les stéroïdes, les inhibiteurs de PDE-IV, les inhibiteurs de MAP kinase p38, les antagonistes de NK₁, les antagonistes de LTD4, les inhibiteurs de EGFR et les antagonistes d'endothéline, ladite autre substance pharmacologiquement active étant administrée simultanément ou successivement.

10. Composition pharmaceutique contenant une indolinone substituée de formule 1 selon l'une quelconque des revendications 1 ou 2 en combinaison avec une autre substance pharmacologiquement active choisie dans le groupe consistant en les agents anticholinergiques, les bêta-2 mimétiques, les stéroïdes, les inhibiteurs de PDE-IV, les inhibiteurs de MAP kinase p38, les antagonistes de NK₁, les antagonistes de LTD4, les inhibiteurs de EGFR et les antagonistes d'endothéline, éventuellement en même temps qu'un ou plusieurs vecteurs ou excipients pharmaceutiquement acceptables.
